# EUROPEAN PATENT APPLICATION

(11) **EP 3 603 680 A1**
(43) Date of publication of application: **05.02.2020**
(21) Application number: 18777095.3
(22) Date of filing: 30.03.2018
(51) Int. Cl.: A61K 49/18

(54) **DIAGNOSTIC IMAGING HEPATIC TISSUE-SPECIFIC CONTRAST MEDIUM COMPRISING MANGANESE SILICATE NANOPARTICLE**

(30) Priority: 31.03.2017 KR 20170041669
(71) Applicant: Postech Academy-Industry Foundation, Pohang-si, Gyeongsangbuk-do 37673 (KR); Research & Business Foundation SungKyunKwan University, Gyeonggi-do 16419 (KR)
(72) Inventor: LEE, Won Jae, Seoul 06502 (KR); LEE, Jung Hee, Seoul 06022 (KR); JANG, Moon Sun, Seoul 06354 (KR); LEE, In Su, Pohan -si Gyeongsangbuk-do 37669 (KR); KIM, Jin Goo, Busan 47516 (KR)
(74) Representative: Agasse, Stéphane
(86) International application number: PCT/KR2018/003810
(87) International publication number: WO 2018/182372

(57) **Abstract**

The present invention relates to a MRI contrast agent for a liver cancer-specific imaging diagnosis including manganese silicate which releases manganese ion (Mn²⁺) under acidic conditions, and a method of characterizing liver tissue using the MRI contrast agent. In a relatively short time, T1-weighted images show different patterns depending on the tissue-specificity such as vascularity, cell density, mitochondrial activity, hepatocellular affinity and the like in normal liver tissues and lesion liver tissues (especially hepatic tumors), and thus, the disease-specific characteristics of liver cancers can be analyzed at appropriate times by analyzing the T1-weighted images. It is expected to be very useful for differentiating the liver cancer types or evaluating the therapeutic effect. Furthermore, it is expected to be useful for diagnosing the diseases occurring in organs other than liver based on the tissue-specificity.

## Description

### [TECHNICAL FIELD]

The present invention relates to a contrast agent for imaging diagnosis of liver tissue, more particularly a contrast agent for a liver cancer-specific imaging diagnosis including manganese silicate which releases manganese ion (Mn²⁺) under acidic conditions, or more specifically a MRI contrast agent for a hepatic tumor-specific imaging diagnosis.

### [RELATED ART]

The role of contrast agent in magnetic resonance imaging (MRI) is used to improve the ability of the diagnosis or differential diagnosis by enhancing the detection ability of the lesion or characterizing the lesion with the contrast enhancement of organs or lesions. Currently, the paramagnetic Gd³⁺ chelate complex based contrast agent is widely used for liver MRI, because it is a nonspecific extracellular fluid (ECF) contrast agent that depends on the difference of vascularity. Among them, Gd-EOB-DTPA is absorbed only in hepatocytes and has hepatobiliary excretion is excreted into the biliary tree, which is called liver-specific contrast agent. However, MR imaging using ECF requires a difficult task such as fast image acquisition and precise start timing. In addition, the possibility of nephrogenic systemic fibrosis due to de-chelated Gd³⁺ ions is to be expected. In addition, the contrast agents such as SPIO (superparamagnetic iron oxide) and MnDPDP (mangafodipirtrisodium) have been developed to overcome the problems of ECF, but they are rarely used due to various problems at present.

SPIO is a nanoparticle preferentially absorbed in Kupffer cells which are one of the cells constituting the liver. On MRI imaging, it serves as a T2 contrast agent, because it reduces the signal intensity in only T2-weighted images in a normal liver including Kupffer cells. It may be the liver-specific contrast agent in an aspect of absorbed in the Kupffer cells. However, it has critical disadvantage of no differential diagnosis for the properties of liver tumors, because it does not affect the signal intensity of liver cancer.

MnDPDP administered intravenously is transmetallated by Zn²⁺ ion in the blood stream, and releases free Mn²⁺ ions. The Mn²⁺ ions in the bloodstream are absorbed by various organs including the liver and increase T1 signal intensity on T1-weighted images. Thus, it is a T1 contrast agent. The MnDPDP absorbed by liver are absorbed in hepatocytes and liver tumors at the same time, but it may appear whiter (high signal intensity) and darker (low signal intensity) depending on the characteristics of liver tumors. Since MnDPDP is also absorbed in hepatocytes, it is not easy to distinguish between the hepatocytes and the liver tumor, resulting in not being gradually used due to the problem of detection for the liver cancer. In other words, MnDPDP has hepatobiliary excretion that is absorbed into hepatocytes and released into the bile ducts. However, it is also absorbed by cells of all organs as well as hepatocytes and is not sufficiently studied. Thus, there has been a limit to call it a liver-specific contrast agent.

The currently used Gd³⁺-based contrast agent has a high accuracy in the detection and characterization of liver caners, but problems such as clinical toxicity of released Gd³⁺ ion (for example, nephrogenic systemic fibrosis) and environmental pollution are emerging. The Mn²⁺-based contrast agent (MnDPDP) capable of complementing to Gd³⁺-based contrast agent has been safe due to a low toxicity, but there were limitations in distinguishing various liver cancers depending on the characteristics of cancers.

Despite the problems of MnDPDP mentioned above, the present inventors have recognized and developed a need for a less toxic T1 contrast agent as compared to a Gd³⁺-based contrast agent (see Korean Patent Publication No. 10-2016-0023963). However, it has disadvantage of relatively long time to obtaining the image of liver cancers because of the slow rate of Mn²⁺ released from Kupffer cells, and has not been studied on the possibility of use for the differential diagnosis according to the characteristics of liver cancers.

Therefore, the MRI contrast agent used for imaging diagnosis must diagnose differentially according to the caner characterization. It is urgent need to develop a liver cancer-specific MRI contrast agent being capable of rapidly acquiring images with this characteristic of differential diagnosis.

### [DETAILED DESCRIPTION OF THE INVENTION]

### [Technical Problem]

The present inventors have concretely found the release and relaxation properties of Mn²⁺ ions and T1-weighted image of the manganese silicate nanoparticles, and have completed the present invention, on the basis of that the manganese silicate nanoparticles releasing manganese ions (Mn²⁺), such as hollow manganese silicate (HMS) nanoparticles or a structure of manganese oxide core and silica shell have a low toxicity, a fast contrasting effect, and an ability to distinguish between liver cancer types through the patterns of different T1-weighted images.

Specifically, MnO core in the nanoparticles including a core of manganese oxide and shell of silica are easily dissolved in a low pH condition (pH 4-5), and since the intracellular condition of the Kupffer cell is in the pH condition, it is useful as pH reactive and liver-specific MRI contrast agent. The contrast agent can enhance the contrast of Kupffer cells located in normal liver tissues against cancerous lesions by releasing Mn²⁺ ions under acidic conditions.

In previous studies of the present inventors, Mn²⁺-doped silica nanoparticles were used as a contrast agent. The hollow manganese silicate (HMS) which is a kind of manganese silicate nanoparticles of the present invention, releases free Mn²⁺ at low pH but their release rate is controlled by the structure of the nanoparticles and the Mn²⁺ release rate of hollow manganese silicate (HMS) is much faster than Mn²⁺-doped silica nanoparticles, thereby exhibiting different T1-weighted MRI imaging of liver in the early stages after intravenous injection of the contrast agent.

In the case of nanoparticles having a manganese oxide core-silica shell structure, the release of Mn²⁺ can be controlled by regulating the thickness and/or porosity of the silica shell in the nanoparticles, The contrast enhancement time can be controlled by regulating the release of Mn²⁺ to the outside of the particle.

The differences in the contrast enhancement patterns of T1-weighted MRI can provide valuable diagnostic information on the pathologic features of liver tumors, and distinguish primary malignant tumors from metastatic tumors and the liver tumor types. The present inventors have found that the optimized release rates of NPs (nanoparticles) designed for lesion detection and characterization were determined, by preparing a series of pH-reactive MnO silica nanoparticles with modulating the rate of Mn²⁺ release at low pH conditions and measuring the various changes in T1-weighted MRI between different tumor models based on pathological features and angiogenesis.

Hereinafter, the present invention will be described in detail.

The present invention relates to an MRI contrast agent for liver tissue containing manganese silicate nanoparticles capable of releasing manganese ions under acidic conditions, wherein the manganese ions are accumulated specifically in the liver tissue, to produce specifically differentiable MRI change pattern.

The manganese silicate nanoparticles are absorbed into Kupffer cells of liver tissues to release manganese ions, and the released manganese ions are excreted outside the Kupffer cells and specifically absorbed and accumulated in the liver tissues to generate a specifically distinguishable MRI change pattern.

The release rate of manganese ions released from the Kupffer cells may be such that the manganese ions are accumulated specifically in the liver tissue and have a release rate to produce MRI change pattern that is specifically distinguished in the liver tissue.

The present invention provides a liver-specific MRI contrast agent comprising manganese silicate nanoparticles, which can specifically distinguish the liver tissue, particularly the kinds and the origins of liver cancers, as well as the liver cancer from normal liver tissue.

The present inventors have found that the release rate and/or release amount of manganese ions in nanoparticles carrying manganese ions endocytosed to Kupffer cells are important in order to distinguish the types and the origins of liver cancers. Specifically, it is necessary for the nanoparticles to release an excess of manganese ion (Mn²⁺) in an instant, in order to specifically distinguish liver tissue.

The contrast agent containing the manganese silicate nanoparticles of the present invention can release manganese ions at a release rate that the manganese ions are specifically accumulated in the liver tissue, to generate a MRI change pattern specifically in the liver tissue.

According to a preferred embodiment of the present invention, the release rate of the manganese ions can be controlled by adjusting the porosity and the layer thickness of the nanoparticles.

According to one embodiment of the present invention, the liver-specific MRI contrast agent may analyze vascularity, cell density, mitochondrial activity, or hepatocellular affinity.

According to an embodiment of the present invention, the MRI contrast agent may be a T1 contrast agent with contrast-enhanced T1-weighted type.

In addition, when the contrast agent is used, it is possible to distinguish liver cancers from normal liver tissues, and the types of liver cancer, and to monitor the therapeutic effect of liver cancers.

The normal liver tissue that can be distinguished by using the contrast agent of the present invention may be a parenchymal tissue, and the types of liver cancers may include all kinds of commonly known liver cancers. Specifically, it may include benign cancers of the liver, primary hepatocarcinoma caused by cancerous mutation of hepatocellular carcinoma, or metastatic hepatocellular carcinoma arising from organs other than liver. The benign tumors include hemangioma, hepatic adenoma and focal nodular hyperplasia (FNH), etc., the primary liver cancers include hepatocellular carcinoma (HCC), cholangiocarcinoma, hepatoblastoma, and angiosarcoma, etc., and the metastatic liver cancers include colonic adenocarcinoma (CAC), and small intestinal neuroendocrine carcinoma (SNC), based on the reference of vascularity.

According to a preferred embodiment of the present invention, the manganese silicate nanoparticles may have a hollow structure.

In the present invention, "hollow manganese silicate (HMS) nanoparticles" have high manganese content and can interrupt and release in a high amount of manganese ions due to the amorphous property of manganese, when exposed to acidic conditions such as phagocyte-endosome circumstance. Therefore, the manganese silicate nanoparticles are easily filtered from the blood and preferentially absorbed by the Kupffer cell, and then released at a large amount of Mn²⁺ ions from the Kupffer cells. The released Mn²⁺ ions are distributed in various liver tissues according to vascularity, cell density, mitochondrial activity, or hepatocellular affinity, and the like of cancers. For examples, the released Mn²⁺ ions are absorbed and accumulated inside the cancers, and thus the nanoparticles have tissue-specificity.

According to an embodiment of the present invention, the manganese silicate nanoparticles may have a manganese oxide core-silica shell structure.

Thereafter, the nanoparticles having the manganese oxide core-silica shell structure are represented by MnO@SiO₂. The silica shells protect the immediate release of Mn²⁺ in a low pH environment and improve the dispersion of particles under neutral pH condition.

The release rate of Mn²⁺ can be controlled by adjusting the thickness and pore size of the silica shell. The release rate decreases as the thickness of the silica shell increases, and the release rate increases as the porosity increases.

The present invention relates to a method for effectively discriminating the types of liver cancers by controlling the thickness and the pore size of the silica shell of manganese oxide core-silica shell nanoparticles. For example, it is possible to provide a nanoparticle with optimal structure to release Mn²⁺ so that the difference of the MRI image change pattern over time between the liver tumor tissue and the normal liver tissue becomes clear or so that the liver cancer can be distinguished within a short time.

The thickness of the silica shell of the manganese oxide core-silica shell nanoparticles is preferably 0.5 nm to 20 nm or 1 nm to 15 nm, or more preferably 2 nm to 12 nm. When the thickness exceeds the upper limit of the thickness range, it has disadvantages of the remarkably reduced release rate of Mn²⁺ and a long MRI measurement time. When it is less than the lower limit thickness, Mn²⁺ is excessively released rapidly, thereby making it difficult to distinguish the types of liver cancers.

According to Example 11 of the present application, it can be seen that the difference of the MRI change pattern over time are generated depending on the types of liver cancers and the time of each type of nanoparticle occurs, and the liver cancers and the liver tumor types can be discriminated efficiently in a short time, by only using a suitable nanoparticle contrast agent with the suitable structure, Detection, and types of liver tumors within a short period of time.

According to a preferred embodiment of the present invention, manganese ions in the manganese silicate nanoparticles of the present invention are contained in an amount of 0.5-55 wt% or 15-55 wt%, or more preferably 19-55 wt% or 20-47 wt%. When the content of manganese ions is included in the nanoparticles, the contrast enhancement of an MRI contrast agent is improved.

According to a preferred embodiment of the present invention, the MRI contrast agent is a pH-responsive signal enhancement type, which is one of the greatest features of the present invention.

More preferably, the hollow manganese silicate (HMS) MRI contrast agent has an r1 value of 0.12 s-1·mM-1, which is specific relaxivities under in vitro neutral condition. That means that does not affect the signal intensity of the MR image, by not reducing the relaxation time of the water proton, in comparison with 7.8 s-1·mM-1 of rl value of free manganese ions. On the other hand, the release of Mn²⁺ ions from HMS is increased in acidic condition, resulting in the contrast enhancement of MRI imaging. According to a preferred embodiment, the acidic condition is pH 2-5.5, or more preferably pH 3-5.

MRI can measure T1 and T2 images by measuring the nuclear spin relaxation of hydrogen molecules in water molecules. MRI contrast agents are divided as T1 contrast agent and T2 contrast agent, and serve to amplify T1 or T2 signals. Each T1 and T2 means spin-lattice relaxation time or spin-spin relaxation time after the nuclear spin excitation in MRI, respectively, resulting in different contrast effects.

According to a preferred embodiment of the present invention, the MRI contrast agent of the present invention is a T1 contrast-enhanced MRI contrast agent. Generally, the manganese ions of paramagnetic substances in the MRI contrast agent of the present invention shows bright or positive contrast effect in comparison of water.

The contrast agent of the present invention can produce a disease-specific change pattern of MRI that changes with time in vivo condition.

The term "specificity" in the present invention means that the MRI contrast agent of the present invention is accumulated or released in a relatively large amount depending on the characteristics of a specific organ or tissue in vivo. The type of the disease of the present invention is not particularly limited, and it may be all diseases in the body organ, preferably a liver disease.

For example, the MRI contrast agents of the present invention have liver-specificity that they are in the large amount in a liver by being firstly absorbed in the form of nanoparticles by Kupffer cells existing only in normal liver tissue. The process of absorption into the hepatocytes in the form of Mn²⁺ cannot be said to be liver-specific, but the process of excretion into the bile duct is liver-specific. From the above, the MRI contrast agent of the present invention can be very usefully applied for diagnosing tumor status and tumor type depending on whether the tumor has liver tumor-specificity, when a liver tumor occurs.

According to another embodiment of the present invention, the MRI contrast agent causes disease-specific signal enhancement in vivo condition.

The disease-specific contrast enhancement is also one of the greatest features of the present invention, and means the tumor region or the tumor margin show the differential contrast enhancement on the basis of the characteristics (blood vessel distribution, cell density, mitochondrial activity, hepatocyte affinity, etc.) of liver tumor types during injection of MRI contrast agent. These characteristics can be used to distinguish the type of liver tumor or to determine the therapeutic effect of the liver tumor by MR imaging.

In one embodiment of the present invention, as a result of preparing HMS and examining the manganese release capacity and T1 contrast effect of HMS, all Mn²⁺ being capable of releasing is released immediately under acidic condition, and the concentration of free Mn²⁺ ions was maximized within 15 minutes (See Example 2). In another example, T1-weighted images were differentiated according to three types of liver tumors (HCC-primary, SNC, CAC-metastasis) (see Example 4).

In another embodiment of the present invention, the nanoparticles having a manganese core-silica shell structure (MnO@SiO₂) are prepared and the thickness and/or pore size of the silica shell is controlled to prepare three types of (MnO@ SiO₂ (HCC-primary, SNC, and CAC-metastatic). When each nanoparticle contrast agent is intravenously injected, the different T1-weighted images are obtained.

Therefore, the nanoparticles of the present invention exhibit different patterns of T1-weighted images depending on the characteristics of liver tumors. In particular, the nanoparticles of the present invention can be used for diagnosing and differentiating the type of liver tumor, or being applied for various purposes and uses required in the determination of therapeutic effect of liver tumor.

According to an embodiment of the present invention, the change pattern of the MRI moving image obtained after administration of MRI contrast agent used for the liver tissue may be a brightness change pattern of the MRI image.

The brightness change pattern can be judged over time when the MRI images photographed along the time are listed.

The contrast enhancement change pattern of the MRI image over the time obtained after administration of the contrast agent may be that that of the normal liver tissue is gradually brightened time-sequentially after administration of the contrast agent and becomes darken after being maximally brightened. Specially, the normal liver tissue may be parenchymal tissue.

According to an embodiment of the present invention, the liver tissue may be hepatocellular carcinoma (HCC). In the change pattern of MRI over time obtained after administering the contrast agent in the normal tissue, the brightness of MRI gradually increases and reaches the maximum brightness, and then becomes dark, as the time goes after administration of the contrast agent. Based on the change pattern of MRI in normal liver tissue, the hepatocellular carcinoma may have a change pattern of MRI that the hepatocellular carcinoma tissue maintains dark state, begins to brighten from the time when the area of normal liver tissue reaches the maximum brightness, and maintains the bright state, as the time goes after administration of the contrast agent.

According to an embodiment of the present invention, the part to be expected as hepatocellular carcinoma shows the change pattern of MRI that the contrast is enhanced inhomogeneously from the peripheral part of tumor right after being injected by HMS, and then is enhanced homogeneously to fill the central part until 24 hours. The part to be expected may be determined as hepatocellular carcinoma.

According to one embodiment of the present invention, in case that the liver tissue is a colonic adenocarcinoma (CAC), the colonic adenocarcinoma tissue maintains black state or darker state than the MRI brightness of the normal liver tissue right after administering the contrast agent, and then brightens gradually or becomes bright circular shape in only peripheral part, on the basis of the change pattern of MRI in normal liver tissue that the brightness of MRI gradually increases and reaches the maximum brightness, and then becomes dark, as the time goes after administration of the contrast agent.

According to an embodiment of the present invention, when the part detected as hepatic tumor maintains the contrast enhancement which is totally relatively reduced from the initial stage to 24 hours after the HMS injection, the specific part may be determined as the CAC.

According to one embodiment of the present invention, in cast that the liver tissue is a small intestinal neuroendocrine carcinoma (SNC), SNC shows the change pattern of the MRI over time obtained after administration of HMS in which the brightness of MRI gradually increases, and is maintained in brighter state than the normal liver tissue, and does not darken, as the time goes after administration of the contrast agent, on the basis of the change pattern of MRI in normal liver tissue that the brightness of MRI gradually increases and reaches the maximum brightness, and then becomes dark, as the time goes after administration of the contrast agent.

According to an embodiment of the present invention, when the part detected as hepatic tumor maintains the contrast enhancement which is totally relatively maintained highly from the initial stage to 24 hours after the HMS injection, the specific part may be determined as the SNC.

According to one embodiment of the present invention, liver cancer can be detected and distinguished by injecting MnO@SiO₂ nanoparticles into each carcinoma (HCC, CAC, SNC) and measuring MRI change with time course. Although the change pattern of MRI occurs in a different time interval depending on the shell thickness and the pore degree of the MnO@SiO₂ nanoparticles, the change pattern of brightness and darkness are common property in the carcinoma.

According to Example 9 of the present invention for HCC, the tissue part in which the high contrast enhancement is maintained for 30 minutes to 1 hour, and low contrast enhancement is maintained for 1 hour to 24 hours, can be determined as a normal liver tissue, in the MRI change pattern taken after the intravenous administration of MnO@5nm-SiO₂ contrast agent. When the part is dark(black) or shows the contrast enhancement in only peripheral region for 30 minutes to 4 hours, and then shows the high contrast enhancement for 4 hours to 24 hours, this part can be determined as HCC tissue.

The tissue part in which the high contrast enhancement is maintained for 30 minutes to 1 hour, and low contrast enhancement is maintained for 1 hour to 24 hours, can be determined as a normal liver tissue.

In case of MnO@8nm-pSiO₂ as contrast agent, when the part shows the high contrast enhancement for 45 minutes to 4 hours and then gradually decreased contrast enhancement for 4 hours to 24 hours, in the MRI change pattern taken after the intravenous administration of MnO@8nm-pSiO₂ contrast agent, the part can be determined as normal liver tissue. In the change pattern of MRI for the normal liver tissue, when the part has the change pattern that the part is in dark (black) for 45 minutes to 4 hours and then the contrast enhancement gradually diffuses from the high enhanced outside to inside of tumor for 4 hours to 24 hours, the part can be determined as HCC tissue.

In case of MnO@10m-SiO₂ as contrast agent, when the part shows the high contrast enhancement for 45 minutes to 8 hours and then contrast enhancement gradually decreases for 8 hours to 24 hours, in the MRI change pattern taken after the intravenous administration of MnO@10nm-SiO₂ contrast agent, the part can be determined as normal liver tissue. In the change pattern of MRI for the normal liver tissue, when the part has the change pattern that the part is in dark (black) or the contrast enhancement is shown in peripheral region for 45 minutes to 8 hours with and then the contrast enhancement is high in the entire tumor region for 8 hours to 24 hours, the part can be determined as HCC tissue.

According to Example 11 of the present invention for CAC(HT29), when the part shows the high contrast enhancement for 15 minutes to 45 minutes, and then gradually decreased contrast enhancement after 1 hour to 24 hours, in the MRI change pattern taken after the intravenous administration of MnO@5nm-SiO₂ contrast agent, the part can be determined as normal liver tissue. In the change pattern of MRI for the normal liver tissue, when the part is in black or is bright in only peripheral region for 15 minutes to 24 hours, the part can be determined as CAC tissue.

When the part shows the high contrast enhancement for 30 minutes to 4 hours, and then gradually decreased contrast enhancement after 4 hour to 24 hours, in the MRI change pattern taken after the intravenous injection of MnO@8nm-pSiO₂ contrast agent, the part can be determined as normal liver tissue. In the change pattern of MRI for the normal liver tissue, when the part is in black or is bright in only peripheral region for 30 minutes to 24 hours, the part can be determined as CAC tissue.

When the part shows the high contrast enhancement for 15 minutes to 4 hours, and then gradually decreased contrast enhancement after 8 hour to 24 hours, in the MRI change pattern taken after the intravenous injection of MnO@10nm-SiO₂ contrast agent, the part can be determined as normal liver tissue. In the change pattern of MRI for the normal liver tissue, when the part is in black or is bright in only peripheral region for 15 minutes to 4 hours, and then shows the contrast enhancement in only the boundary region of tumor after 8 hours to 24 hours, the part can be determined as CAC tissue.

According to Example 11 of the present invention for SNC (STC-1), in case that two parts cannot be distinguished for 15 minutes to 1 hour but one part becomes bright due to the gradual contrast enhancement after 4 hours to 24 hours, in the MRI change pattern taken after the intravenous injection of each contrast agent of MnO@5nm-SiO₂, MnO@8nm-pSiO₂, and MnO@10nm-SiO₂, the part can be determined as SNC tissue.

According to a preferred embodiment of the present invention, the MRI contrast agent causes signal intensity enhancement *in vivo* in sequential order of time. More specifically, when the HMS contrast agent is administered, it exhibits high signal intensity in the normal liver tissue at 0.1 to 5 hours after *in vivo* injection, and exhibits high signal intensity in liver disease tissue at 6 to 24 hours after in vivo injection.

This characteristic of signal enhancement in time difference is one of the greatest features of the present invention, because HMS adsorbed by Kupffer cells in normal liver tissue releases Mn²⁺ ion in an acidic endosome surroundings, so as to exhibit the high signal intensity in the normal liver tissue but the low signal intensity in the liver lesion tissue up to 5 hours after injection of HMS contrast agent. Then, because the Mn²⁺ ions released and diffused from outside of Kupffer cell are absorbed by the liver lesion tissue, the low signal intensity is exhibited in the normal liver tissue but the high signal intensity is shown in the liver lesion.

In other words, normal liver tissue appears whitish and then becomes black in T1-weighted images, but the hepatic lesion tissue appears black and then becomes whitish.

Due to this signal enhancement in time difference, the double MRI picture showing clearly the hepatic lesion tissue can be collected at a mode of reverse contrast in a session of single MRI picture, and the detection rate of the hepatic lesion tissue and the possibility of differential diagnosis can be increased.

An embodiment of the present invention is to provide a method of characterizing the hepatic tissue, comprising administering to a subject at least one of the contrast agents; obtaining a magnetic resonance image obtained by continuously imaging liver tissue of the subject over time; and extracting a temporal change pattern of the magnetic resonance imaging for liver tissue.

The change pattern over time of the magnetic resonance imaging for liver tissue due to the characteristics of the liver tissue is the same as described in the above section of the contrast agent.

In addition, an embodiment of the present invention provides a method of providing an information on diagnosis of liver tumor and determination of liver tumor type, comprising administering to a subject at least one of the contrast agents; obtaining a magnetic resonance image obtained by continuously imaging liver tissue of the subject over time.

The information on diagnosis of liver tumor and determination of liver tumor type can be the brightness change pattern of magnetic resonance imaging over time.

The subject may be an animal, preferably a mammal, and may exclude a human.

### [EFFECTS OF THE INVENTION]

The present invention relates to a MRI contrast agent for liver tissues comprising manganese silicate nanoparticles which release manganese ions (Mn²⁺) under acidic conditions. The present invention relates to a MRI contrast agent for liver tissue, which is capable of inhibiting vascularity and cell density. The pattern of T1-weighted images is different, depending on the tissue characteristics of liver tumors such as vascularity, cell density, mitochondrial activity, hepatocellular affinity, and the like. By analyzing the T1-weighted images, the characteristics of liver tumors can be detected in a short time, and thus, it is expected to be very useful for differentiating the types of liver tumors and determining the therapeutic effect.

### [BRIEF DESCRIPTION OF THE DRAWINGS]

Fig. 1 shows the manganese release capacity and T1 contrast enhancement effect of hollow manganese silicate (HMS) nanoparticles, a) TEM image of synthesized nanoparticle, b) TEM image of pH 5 citrate buffer suspension, c) the amount of Mn²⁺ released from HMS particle, and d) T1-weighted images.
Fig. 2 is a schematic diagram of T1-weighted image and function mechanism after intravenous injection of HMS (3 mg / kg) into a hepatocellular carcinoma (HCC) model.
Fig. 3a shows T1-weighted images of representative three types of liver tumors (primary-HCC, metastatic-SNC (small intestinal neuroendocrine carcinoma), and CAC (colonic adenocarcinoma)), specifically the imagens for a) HCC model, b) SNC model, and c) CAC model image, and Fig. 3b shows the detailed pictures.
Fig. 4 shows the distribution and activity of cells by staining mitochondria according to the anti-prohibin antibody staining (brown coloring), and the cell density and the vesicular distribution of hepatic tumor types by staining nuclei of cells according to hematoxylin staining (blue coloring).
Fig. 5 is a graph showing contrast enhancement effect on vascular distribution for three kinds of liver tumors through Fast spin echo T1-weighted image.
Fig. 6 is a graph showing the T1-weighted image obtained at the HAO (hepatic artery occlusion) (upper) and HCC necrosis (lower) after hepatic artery ligation using NADH-tetrazolium reductase staining.
Fig. 7 is schematic diagrams, microscopic images and the ingredients and their amount used for synthesis of MnO@5nm-SiO₂ (MnO-SiO₂-0.5hr), MnO@8nm-pSiO₂ (MnO-SiO₂-2hr), MnO@10nm-SiO₂ (MnO-SiO₂-12hr) of nanoparticles.
Fig. 8a is a graph showing the perspective view of MnO@5nm-SiO₂ (MnO-SiO₂-0.5hr), MnO@8nm-pSiO₂ (MnO-SiO₂-2hr), MnO@10nm-SiO₂ (MnO-SiO₂-12hr) nanoparticles respectively, and the increased releasing rate of Mn²⁺ in a sequential order of MnO-SiO₂-0.5h, MnO-SiO₂-2h and MnO-SiO₂-12h.
Fig. 8b is a graph showing the releasing rate of Mn²⁺ ions from MnO @10nm-SiO₂ (MnO-SiO₂-0.5 hr) under the conditions of pH 5, pH 6 or pH 7 with time passage.
Fig. 8c is a graph showing the releasing rate of Mn²⁺ ions from MnO@8 nm-pSiO₂ (MnO-SiO₂-2 hr) under the conditions of pH 5, pH 6 or pH 7 with time passage.
Fig. 8d is a graph showing the releasing rate of Mn²⁺ ions from MnO@5 nm-SiO₂ (MnO-SiO₂-0.5 hr) under the conditions of pH 5, pH 6 or pH 7 with time passage.
Fig. 8e is a graph showing the comparison of the releasing rate of Mn²⁺ ions for MnO@10nm-SiO₂ (MnO-SiO₂-12hr), and MnO@8nm-pSiO₂ (MnO-SiO₂-2hr), MnO@5nm-SiO₂ (MnO-SiO₂-0.5hr)
Fig. 9a is a graph showing the change of contrast intensity of T1-weighted images obtained by Mn²⁺ ions released from MnO@10nm-SiO₂ (MnO-SiO₂-12hr), MnO@8nm-pSiO₂ (MnO-SiO₂-2hr), MnO@5nm-SiO₂ (MnO-SiO₂-0.5hr) nanoparticles.
Fig. 9b is a graph showing the numeric values by quantifying the contrast intensity of the image photographed in FIG. 9a (R1, unit = second).
Fig. 9c is a graph showing the relationship between the concentration of Mn²⁺ ions and the contrast intensity (R1) of the image.
FIG. 10a shows MRI photographed before the injection, and 15 minutes, 30 minutes, 45 minutes, 1 hour, 4 hours, 8 hours or 24 hours after intravenous injection of MnO@5nm-SiO₂ (MnO-SiO₂-0.5 hr) nanoparticles into the HCC tumor model.
FIG. 10b shows MRI photographed before the injection, and 15 minutes, 30 minutes, 45 minutes, 1 hour, 4 hours, 8 hours or 24 hours after intravenous injection of MnO@8nm-pSiO₂ (MnO-SiO₂-2 hr) nanoparticles into the HCC tumor model.
FIG. 10c shows MRI photographed before the injection, and 15 minutes, 30 minutes, 45 minutes, 1 hour, 4 hours, 8 hours or 24 hours after intravenous injection of MnO@10nm-SiO₂ (MnO-SiO₂-12 hr) nanoparticles into the HCC tumor model.
Figs. 11a to 11e are graphs showing the results of measuring SNR (signal-to-noise ratio) values of the contrast enhancement effects over time for each organ after the injection of MnO@10nm-SiO₂ (MnO-SiO₂-0.5hr), MnO@8nm-pSiO₂ (MnO-SiO₂-2hr), MnO@5nm-SiO₂ (MnO-SiO₂-0.5hr) nanoparticles to each organ: Fig. 11a for hepatic tumor, Fig. 11b or normal liver tissue, Fig. 11c for kidney, Fig. 11d for spleen, and Fig. 11e for intestine.
Fig. 12a is MRI photographed before the injection, and 15 minutes, 30 minutes, 45 minutes, 1 hour, 4 hours, 8 hours or 24 hours after intravenous injection of MnO@5nm-SiO₂ (MnO-SiO₂-0.5 hr) nanoparticles into the CAC tumor model.
FIG. 12b shows MRI photographed before the injection, and 15 minutes, 30 minutes, 45 minutes, 1 hour, 4 hours, 8 hours or 24 hours after intravenous injection of MnO@8nm-pSiO₂ (MnO-SiO₂-2 hr) nanoparticles into the CAC tumor model.
FIG. 12c shows MRI photographed before the injection, and 15 minutes, 30 minutes, 45 minutes, 1 hour, 4 hours, 8 hours or 24 hours after intravenous injection of MnO@10nm-SiO₂ (MnO-SiO₂-12 hr) nanoparticles into the CAC tumor model.
Fig. 13a is MRI photographed before the injection, and 15 minutes, 30 minutes, 45 minutes, 1 hour, 4 hours, 8 hours or 24 hours after intravenous injection of MnO@5nm-SiO₂ (MnO-SiO₂-0.5 hr) nanoparticles into the SNC tumor model,
FIG. 13b shows MRI photographed before the injection, and 15 minutes, 30 minutes, 45 minutes, 1 hour, 4 hours, 8 hours or 24 hours after intravenous injection of MnO@8nm-pSiO₂ (MnO-SiO₂-2 hr) nanoparticles into the SNC tumor model,
FIG. 13c shows MRI photographed before the injection, and 15 minutes, 30 minutes, 45 minutes, 1 hour, 4 hours, 8 hours or 24 hours after intravenous injection of MnO@10nm-SiO₂ (MnO-SiO₂-12 hr) nanoparticles into the SNC tumor model.

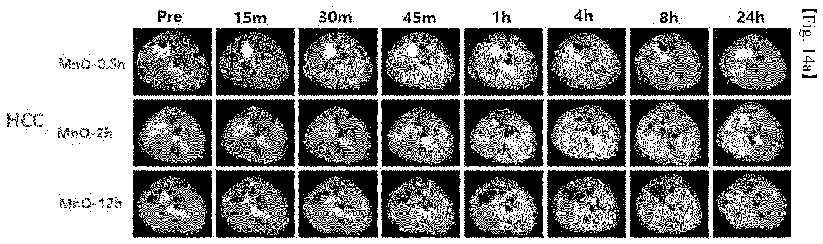

Fig. 14a shows the MRI contrast images collected by Figs. 10a to 10c.

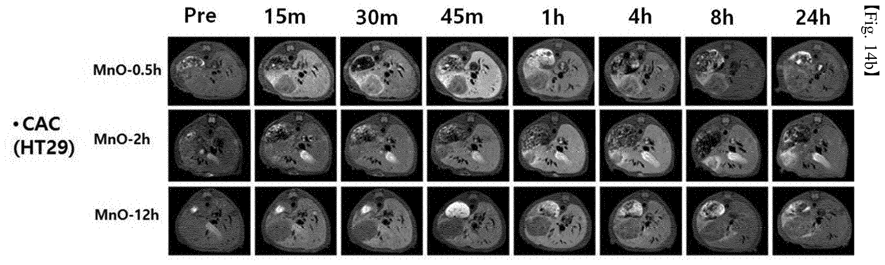

Fig. 14b shows the MRI contrast images collected by Figs. 12a to 12c.
Fig. 14c shows the MRI contrast images collected by Figs. 13a to 13c.
Fig. 15a shows the changes in MRI contrast image with time passage after administration of MnO@5nm-SiO₂ (MnO-SiO₂-0.5 hr) contrast agent to each of three tumor models.
Fig. 15b shows the changes in MRI contrast image with time passage after administration of MnO@8nm-pSiO₂ (MnO-SiO₂-2 hr) contrast agent to each of three tumor models.
Fig. 15c shows the changes in MRI contrast image with time passage after administration of MnO@10nm-SiO₂ (MnO-SiO₂-12hr) contrast agent to each of three tumor models.

### [DETAILED DESCRIPTION OF THE INVENTION]

Hereinafter, preferred embodiments of the present invention will be described in order to facilitate understanding of the present invention. However, the following examples are provided only for the purpose of easier understanding of the present invention, and the present invention is not limited by the following examples.

### [EXAMPLE]

### Example 1. Preparation of materials and procedure

### 1-1. Experimental Materials

MnCl₂•4H₂O (kanto), Sodium Oleate (TCI), 1-octadecene (Aldrich), Igepal® CO-520 (Aldrich), Tetraethyl Orthosilicate (Acros), NH₄OH (Samchun Chem.), Ni(NO₃)6H₂O (Strem), Cu(NO₃)₂·3H₂O (Strem), NaBH₄ (Samchun Chem.), 2-[methoxy(polyethylenoxy)-propyl]-9-12-trimethoxysilane (MPEOPS, Gelest, Inc.), fluorescein isothiocyanate (FITC, Aldrich), and 3-aminopropyltriethoxysilane (APTES, Aldrich) were used as purchased without any purification.

### 1-2. Preparation of HMS (hollow manganese silicate)

According to previously reported technique, HMS (hollow manganese silicate) nanoparticles (NPs) was synthesized by annealing MnO@SiO₂/Cu²⁺ and then, selectively etching outer silica shell of Cu@HSNP (hollow-structured NPs) (Kim, J. G.,Kim, S. M. & Lee, I. S. Mechanistic insight into the yolk@shell transformation of MnO@silica nanospheres incorporating Ni2+ ions toward a colloidal hollow nanoreactor. Small 11, 1930-1938 (2015)). The synthesized HMS has the following properties; HMS (39±2 nm) including an encapsulated Cu nanocrystal(9±1 nm) inside of hollow pore (14±2 nm) which was synthesized from nanocrystal containing Cu²⁺-bound SiO₂ nanosphere according to solid-state-hollowing.

### 1-3. Release and relaxation properties of HMS

The release profile of manganese ions from HMS was quantified at room temperature using 8 mg of HMS suspensions in 16 ml of citrate buffer at pH 5 and pH 6.2 and distilled water. 4 ml of each HMS suspensions was collected from samples at 15 minutes, 1 hour, 7 hours and 20 hours, and the nanoparticles (NPs) of the sample were removed by centrifugation at 15,000 rpm for 10 minutes, and each supernatant was filtered by using syringe filter with 0.2 mm of pore size. The amount of each released manganese ion and the T1 relaxation time of the proton in each supernatant were quantified using ICP-AES and a 3.0-T clinical MR scanner (Philips, Achieva ver.1.2, Philips Medical Systems, Best, The Netherlands).

### 1-4. Preparation of animal models (primary-HCC, metastatic-SNC and CAC)

6-week-old BALB/C nude mice (male of HCC model and female of metastatic carcinoma model) were purchased from Orient Bio (Seoul, Korea). All animal studies were approved by the Animal Laboratory Use Committee of the Samsung Life Sciences Institute. The HCC model was constructed using a human HCC cell line (HepG2, Korean Cell Line Bank) and the metastatic carcinoma models were constructed using human CAC cell line (HT29, ATCC) and mouse SNC cell line (STC-1, ATCC). The cell lines were maintained in Dulbecco's Modified Eagle's medium (STC-1) containing minimal essential medium (HepG2), McCoy 5a medium (HT29) and 10% fetal bovine serum (Invitrogen) and 1% antibiotic (ThermoFisher). The cells were cultured at 37 ° C in 5% CO₂ and recovered with 0.25% of Trypsin / EDTA (ThermoFisher). The recovered cells (1x10⁶ HepG2, 5x10⁶ HT29, and 1x10⁷ STC-1) were suspended in 10 µl HBSS containing Matrigel (1: 1). After sampling the cells, the mice were completely anesthetized by applying 2% (v/v) isoflurane via a face mask as a mixture of O₂ / N₂ gas (3: 7 ratio) and exposing the liver to surgery. The mixed cells with Matrigel were slowly injected into the liver and the incision was blocked. After 4 to 6 weeks, MR images were used to confirm tumor size.

### 1-5. MRI Imaging

MR images of HCC, SNC and CAC tumors were obtained by the following method. The animals were anesthetized by administering 5% (v/v) isoflurane mixed with O₂ / N₂ gas (3: 7 ratio) through a face mask, and maintained by administering 1.5-2% isoflurane.

The body temperature was maintained at 36 ± 1 ° C using a circulating water warming pad and respiratory rate was continuously monitored during the entire scan time. After obtaining the pre-contrast MRI images before the contrast enhancement, HMS (Mn 3 mg per kg of body weight) was delivered with intravenous injection via the tail vein. The post-contrast MRI images after the contrast enhancement were collected at 3 minutes, 6 minutes, 9 minutes, 15 minutes, 30 minutes, 1 hour, 3 hours, 6 hours, 12hours, 24 hours and 48 hours.

Furthermore, MR images of the brain and various organs were obtained from mice with HCC tumor. After obtaining pre-contrast MR images, intravenous injection of HMS (Mn 3 mg / kg body weight) through the tail vein was performed to obtain MR images.

All *in vivo* MR imaging was performed using a 7 T / 20 MR system (Bruker-Biospin, Fallanden, Switzerland) with a 20-cm tilt set capable of delivering up to 400 mT / m at a 100-µs rise-time. First, the MR images of the mice liver were used for excitation and signal reception by a quadrature volume coil (35 mm id), and a fast spinecho (FSE) T1-weighted MRI sequence ((repetition time TR) / echo time (TE) = 380 / 7.7 ms, experiment number NEX = 3, echo train length = 2, in-plane resolution 200 x 200 mm, slice thickness 1 mm and 14 slices). After 30 minutes of HMS injection, the FSE T1-weighted MRI sequence (TR / TE = 380 / 7.7 ms, NEX = 8, echo train length = 2, in-plane resolution 100 x 100 mm, slice thickness 1 mm and 14 slices) were used to re-measure MR images.

To obtain brain MR images, a birdcage coil (72 mm i.d.) was used for excitation and an actively-decoupled phased array coil was used for signal reception. Brain imaging was performed on an FSE T1-weighted MRI sequence (TR / TE = 325 / 7.7 ms, NEX = 12, echo train length = 2, in-plane resolution 100 x 100 mm, 12 slices).

### 1-6. Immunostaining (Anti-prohibin antibody)

The liver tissue was extracted from cancer animal model and was immediately fixed on 10% formalin solution. The fixed tissue was prepared to paraffin block, cut to 4 µm thickness and fixed on slide. The tissues fixed on the slide were deparaffinized and then stained with antiprohibitin (abcam, mitochondrial markers) and hematoxylin (nuclei staining). After staining, the stained tissue was mounted on a staining-resistant mounting medium and visualized using an Olympus DP70 digital fluorescence microscope camera (Olympus, Melville, NY).

### 1-7. NADH-tetrazolium reductase staining

The liver tissue was extracted from tumor animal model and was immediately buried in Optimal Cutting Temperature compound (OCT compound, Tissue Tek, Sakura, France) and placed in liquid nitrogen-cooled isopentane until incision. The frozen samples were cut to 5 µm of size using cryostat (Thermo Scientific, Kalamazoo, MI), were dropped by 0.4 mg / ml NADH and 0.8 mg / ml NBT (4-nitro blue tetrazolium chloride), and then were reacted at 37 °C for 30 to 60 minutes. After washing, the samples were mount on the staining-resistant mounting medium and visualized in the same manner as Example 1-6.

### Example 2. Mn relaxation property and T1 contrast enhancing property of Hollow manganese silicate (HMS) nanoparticle

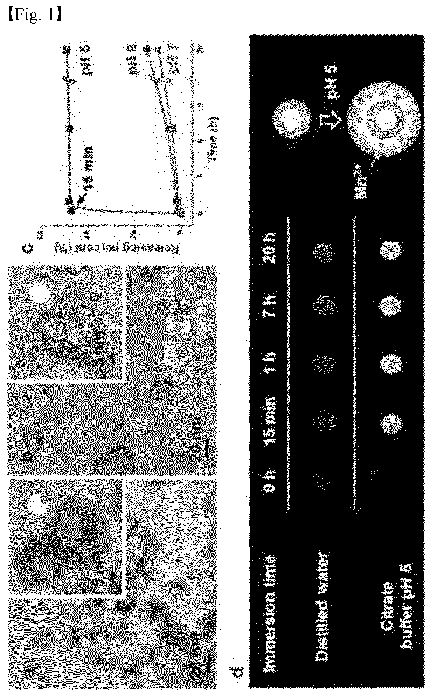

The Mn release property and T1 contrast enhancing property of HMS prepared in Example 1-2 were tested according to Example 1-3. The synthesized nanoparticle and the nanoparticle separated from a buffer solution at pH 5.0 were observed with TEM (a and b in Fig. 1).

As a result of c and d in Fig. 1, when PEG-modified HMS containing 19.3 wt% of Mn was immersed in a buffer solution at pH 5.0, all Mn²⁺ being capable of releasing released immediately, and increased to the maximum concentration of free Mn²⁺ ion within 15 minutes. In contrast, when the PEG-modified HMS was immersed in buffer solution or distilled water at pH 6.0, Mn²⁺ ions were released very slowly (Fig. 1c). In addition, the T1-weighted image in distilled water did not affected by HMS. However, when HMS was added to buffer solution at pH 5.0, T1 signal intensity increased very shortly, and thus, T1-weighted image showed brightly after 15 minutes of adding HMS.

These results indicate that the contrast enhancement effect of Mn²⁺ efflux from HMS is remarkable in acid solution.

### Example 3. Evaluation on the contrast enhancement pattern of HMS in HCC

Firstly, HMS was injected (3 mg / kg body weight) into a human hepatocellular carcinoma (HCC) model and observed for 0.5-24 hours after HMS injection to evaluate the possibility of HMS as a contrast agent for distinguishing liver tumors.

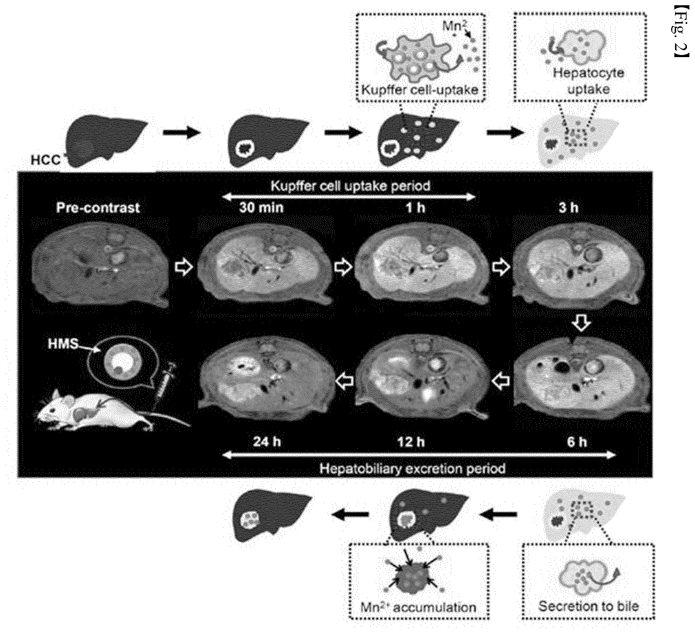

As a result of FIG. 2, the significant contrast enhancement was observed for 0.5-6 hours after the injection of HMS, and then it slowly began to brighten from the periphery region to the center region for 6-24 hours, and the necrotic area was not enhanced.

It can be interpreted that these results demonstrate that Mn²⁺ ions were released from the nanoparticles absorbed in Kupffer cells during the first 0.5-6 hours (Cooper cell uptake period) and then Mn²⁺ ions released into liver tissue were absorbed by liver cells and released into the bile for 6-24 hours (bile release period), that is, ions are absorbed by hepatocytes and then released into the bile.

### Example 4. Characterization of the tissue properties of liver tumor after the contrast enhancement by HMS in T1-weighted image (Distinguishable diagnosis effect depending on the vascularity, cell density, mitochondria activity and hepatocellular affinity)

On the basis of the result in Example 3, three kinds of liver tumors models prepared in Example 1-4 (primary-HCC, metastatic-SNC and CAC) were tested for the difference in the contrast enhancement patterns of T1-weighted image.

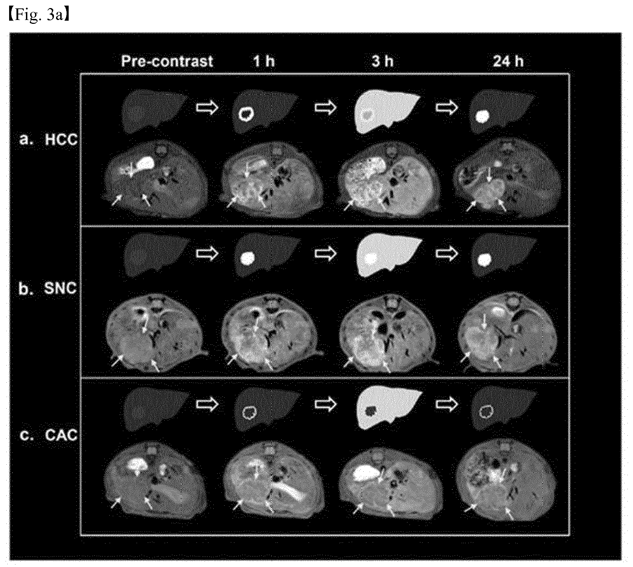

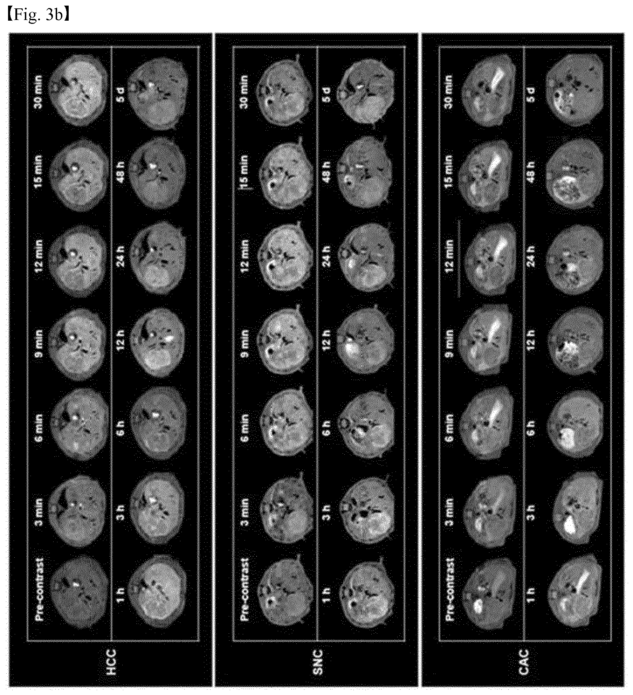

As a result of Fig. 3a, during 24 hours after HMS injection, the HCC model showed the gradual enhancement of signal intensity of tumor up to 24 hours from the initial stage. The small intestinal neuroendocrine carcinoma (SNC) model showed that the signal intensity of the tumor remained strong until 24 hours from the beginning. The CAC (colonic adenocarcinoma) showed that the signal intensity of the tumor remained weak until 24 hours from the beginning. Fig. 3b shows a similar pattern up to 24 hours after injection of HMS. More specifically, the HCC model showed that the contrast enhancement became heterogeneously from the periphery of the tumor in 1 hour after the injection of HMS, and gradually filled to the center region of the tumor being accompanied with homogeneous enhancement until 24 hours. In the SNC model, the contrast enhancement degree of the peripheral portion of the tumor was higher than that of the central portion, but the enhancement area was larger than that of the HCC model. In contrast to the HCC model, the contrast enhancement of the tumor in SNC model was maintained strongly to 24 h, although the contrast enhancement of the tumor was slightly increased compared to HCC model. Finally, the CAC model was observed for 1 hour to 24 hours after HMS injection. The contrast enhancement degree of the tumor in CAC model was maintained as being significantly reduced state compared to the HCC or SNC model, and showed a thin band-like contrast enhancement in the surrounding area of the tumor. As a result, in all three liver caners, the degrees of contrast enhancement of the tumor were decreased after 24 hours and the contrast enhancement in liver parenchyma was most strongly increased after 1-3 hours, as shown in the images after 48 hours and 5 days.

As a result, the Gd³⁺ based MRI contrast agent which is currently used mainly is a contrast agent used for dynamic study in which blood flow distribution is simply observed within a short time after injection of contrast agent. On the other hand, the results of this experiment showed that the contrast agent using HMS in the present invention shows a very different contrast enhancement image observed depending on the distribution of manganese ions in the tumor over 24 hours after injection into liver tumor.

Namely, HMS is expected to have an excellent effect on the differential diagnosis of liver tumors, because it has a high possibility of obtaining characteristic contrast enhancement pattern depending on the tissue characteristics as well as the blood flow distribution. In this aspect, if HMS-enhanced MRI imaging provides characteristic features in each tumor, HMS can be called as a disease-specific MRI contrast agent.

### Example 5. Distinguishable diagnosis effect depending on the tissue characteristics (vascular distribution, cell density, mitochondria activity and hepatocellular affinity) of liver tumor by using mitochondria staining

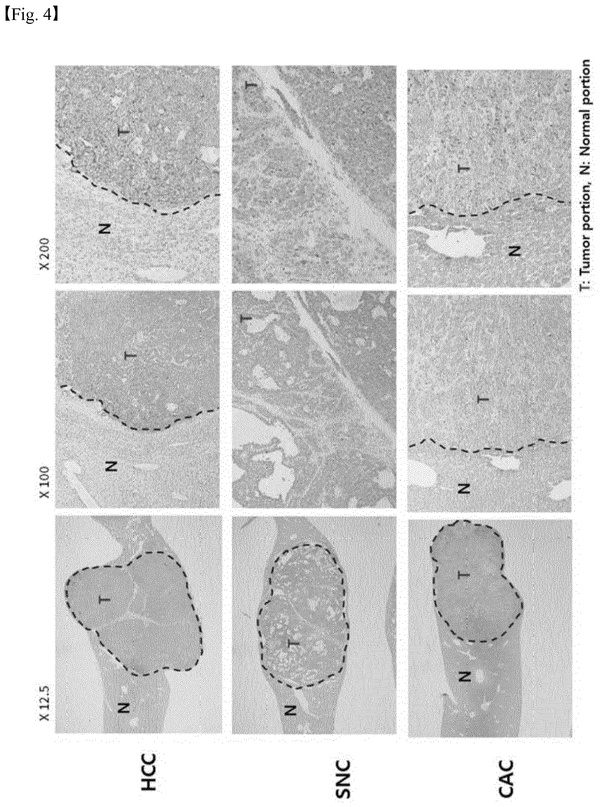

Manganese ions are deposited in mitochondria present in all cells. Therefore, if the mitochondrial activity is high in certain organs or diseases, manganese ions released from HMS migrate to the organs or diseases and are massively deposited, thereby exhibiting the high signal intensity in T1-weighted image. Based on the results of Examples, in order to test whether the MRI obtained by the contrast enhancement of HMS reflects the tissue specificity of disease, the mitochondria activity was measured and shown in Fig. 4.

First, according to the distribution of mitochondria, the HCC model shows higher staining degree in tumor than in liver parenchyma, whereas the CAC model shows higher staining degree in liver parenchyma than in tumor. In this case, the region with higher staining degree includes more mitochondria. There are more mitochondria in some tissues, which means the higher number of mitochondria in the cells of the tissues or the higher density of the cells in the tissues.

In addition, as a result of analyzing the vascular distribution and cell density by hematoxylin staining (cell nuclei staining) and antibody staining, HCC cell density was higher than that of liver parenchyma in the HCC model. In the CAC model, much cell necrosis occurred in the central region of CAC, and the cell density at the peripheral region was lower than that of the liver parenchyma.

In the HCC model, more mitochondria were present in the HCC than in the liver parenchyma, and T1-weighted images showed higher signal intensity in the HCC than in the liver parenchyma. Conversely, in the CAC model, more mitochondria were present in the liver parenchyma than in CAC, and T1-weighted images showed lower signal intensity in the CAC than in the liver parenchyma. In the CAC model, the contrast enhancement in a band shape was presumed that the cell density was high in the region due to the compact of tumor cells or surrounding normal cells in compression.

Finally, in the SNC model, although the staining degree in the liver parenchyma and tumor is not high with no significant difference, the tumor showed a very high degree of contrast enhancement compared to the liver parenchyma. This can be presumed that the SNC model is a hypervascular tumor with very high vascular distribution in mitochondrial staining.

In addition, the HCC model shows the increased contrast enhancement over time, but the SNC model maintains a relatively constant high contrast enhancement. The concentration of manganese ions increases gradually in the HCC model, because the manganese ions are absorbed into the cells but discharged into biliary tract due to the maintenance of hepatocellular affinity (Ni, Y, et al., Tumor models and specific contrast agents for small animal imaging in oncology, Methods 48, 1930-1938 (2015)). In the SNC model, a large amount of manganese ions are accumulated from the initial stage, because the manganese ions are only absorbed into the cells, and are not discharged into the biliary ducts due to no hepatocellular affinity.

Thus, three types of liver tumors have various tissue characteristics such as vascular distribution, cell density, mitochondrial activity, and hepatocellular affinity of each liver tumor, thereby providing the three types of liver tumors with the characteristic contrast images at specific imaging time in T1-weighted images.

### Example 6. Liver cancer-specific vascularity by using FSE Tl-weighted image

On the basis of the above results, the fast spin echo (FSE) T1-weighted image which was obtained from 3 minutes to 15 minutes after HMS injection showed the contrast enhancement effect on three types of liver cancers.

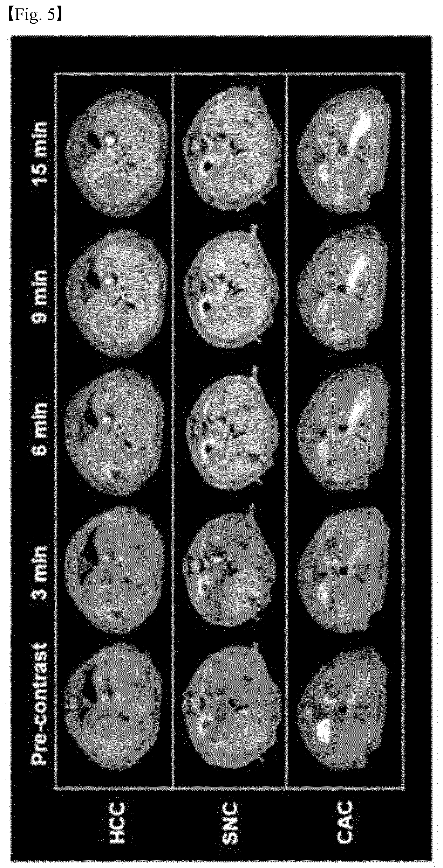

As a result of Fig. 5, these results indicate that HMS is mainly absorbed by Kupffer cells at this stage (3 ∼ 15 min after HMS injection), and thus the contrast enhancement pattern appears more rapidly at the contrast enhancement time of liver tumor, in which the contrast enhancement of the liver tumors is occurred by releasing the manganese ions. Therefore, the contrast enhancement caused by the vascular distribution reflects more than the contrast enhancement caused by the mitochondrial activity at the initial stage.

More specifically, the contrast enhancement pattern of the tumor in the HCC model and the SNC model at this stage was more distributed in the periphery region than the image obtained after 1 hour after HMS injection. However, in the CAC model, only a weak contrast enhancement in a band shape was observed and the tumor itself did not show the contrast enhancement.

As a result of the above results, it is expected that the vascular distribution can contribute to the enhancement patterns of the three types of liver tumors, which can be used for differential diagnosis of the liver tumors.

### Example 7. Evaluation of necrosis (cell death) degree of HCC after hepatic artery ligation by NADH-tetrazolium reductase staining

Among the methods of treatment of liver cancer, TACE is a treatment method of necrosis of HCC by blocking blood flow to the tumor with blocking the patient's hepatic artery. In order to confirm whether there is a correlation between tumor necrosis (cell death) and HMS-signal intensities of T1-weighted images with time after hepatic artery ligation of the HCC model mice, NADH-tetrazolium reductase staining method was used for measuring the NADH-dehydrogenase activity of the intracellular mitochondria in the live cells (Berardi et al., Neurol Res. 2008; 30 (2): 160-9).

More specifically, the abdominal wall of an HCC model mouse was dissected and left hepatic artery was ligated and the abdominal wall was closed. HMS was administered to the animal after 2 hours and 24 hours. After 2 hours and 24 hours from the HMS injection, MRI images were obtained.

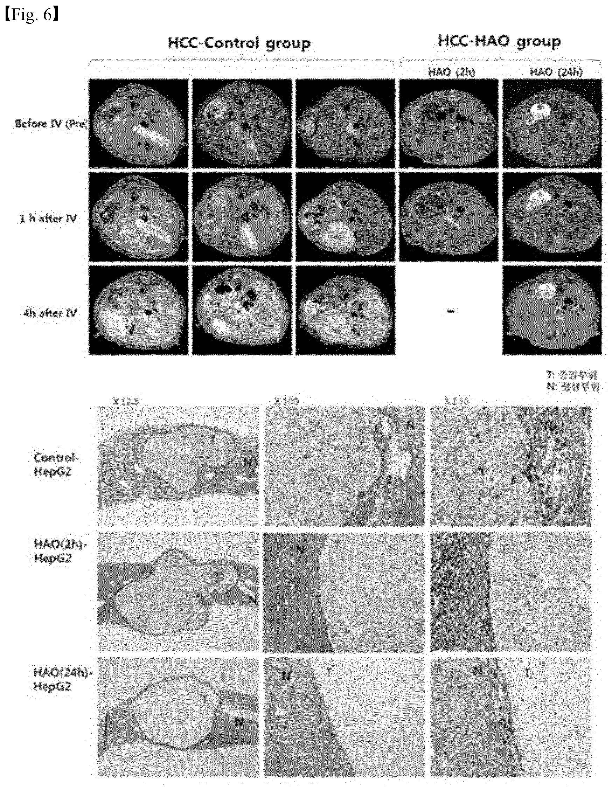

As a result shown in FIG. 6, T1-weighted images obtained after 1 hour of injecting HMS at 2 hours elapsed from the ligation confirmed that the tumor exhibited the contrast enhancement in the periphery. Because the tumor cells were stained overall with weaker degree than the control group, the survived tumor cells were found. Therefore, by combining the staining results and the MRI results, because the blood flow is supplied to the tumor through the hepatic portal vein in the hepatic artery ligation, and the tumor cells surviving in only region supplied with the blood were stained with NADH staining, and the same region shows the contrast enhancement in T1-weighted images.

In addition, in T1-weighted images obtained after 1 hour of injecting HMS at 1 hour and 4 hours elapsed from the ligation, the contrast enhancement was not observed in the tumor at all, and no cell stained with NADH staining confirmed the complete necrosis of tumors.

As a result of summarizing the results, it is expected that the mitochondrial activity of HMS can be used to evaluate the cancer cell necrosis (cell death) of HCC, thereby being used for determination of the therapeutic effect by evaluating the cell necrosis after anti-cancer therapy as well as TACE.

The foregoing description of the invention is illustrative. It will be appreciated by those skilled in the art that the present invention may be modified in other specific forms without departing from the spirit or essential characteristics of the invention.

### Example 8. Preparation of MnO@SiO₂ NPs as a MRI contrast agent

### 8-1. Preparation of MnO@10nm-SiO₂ (MnO-SiO₂-12hr) and measurement of Mn²⁺ releasing time

### (1) Preparation of MnO@10 nm-SiO₂ (MnO-SiO₂- 12 hr) nanoparticles

MnO NPs were used as the Mn²⁺ ion source. These particles are not dissolved in the neutral pH condition (∼ pH 7) and dissolved in the low pH condition (pH 4 ∼ 5).

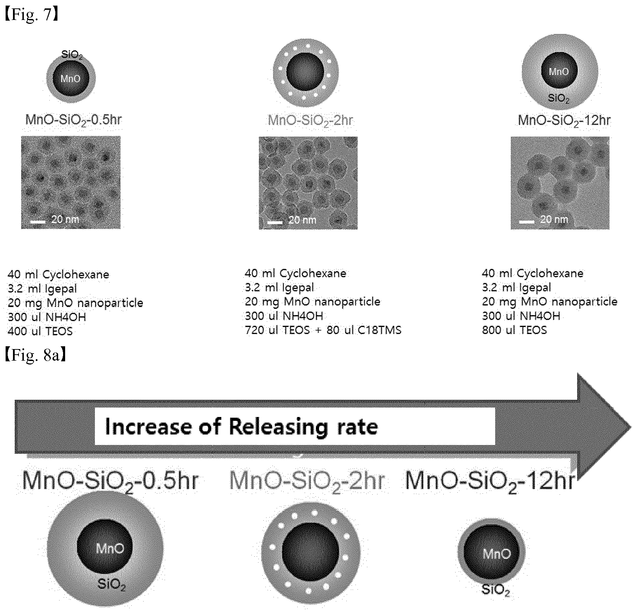

First, to reduce the release rate of Mn²⁺ from the particles, MnO NPs (15 nm) were synthesized, and the synthesized MnO NPs (15 nm) were covered with silica shell using a reverse-micelle emulsion template (FIG. 7).

To synthesize 15 nm MnO nanoparticles (NPs), 1.24 g of Mn-oleate complex was dispersed in 10 g of 1-octadecene solvent, and the solution was heated to 300 ° C at a rate of 5°C/minute with stirring under the blocking condition of oxygen and moisture. Then, the solution was heated at 300 ° C for 1 hour, cooled to room temperature, and centrifuged to separate the particles. The separated particles were washed with hexane and acetone, and the synthesized MnO nanoparticles with 15 nm in size were dispersed in cyclohexane and stored.

Next, MnO@SiO₂ nanoparticles were obtained by surrounding the MnO nanoparticles with a silica shell using a reverse-micelle emulsion template. Specifically, after 20 mg of MnO nanoparticles were dispersed in 40 mL of cyclohexane, 3.2 mL of a surfactant, IGEPAL CO-520 was added, and the mixture was stirred for 30 minutes. Thereafter, 0.3 mL of NH₄OH solution and 0.8 mL of TEOS (Tetraethyl orthosilicate) were added in order at intervals of 30 minutes. After the solution was stirred for one day, 5 mL of methanol was added to separate an aqueous layer and an organic layer. The aqueous layer was separated and centrifuged to separate the particles, and the particles were washed with ethanol and water to obtain MnO@10 nm-SiO₂ particles surrounded by silica shell of 10 nm thickness.

### (2) Measurement of Mn²⁺ release time

The silica shells prevent the immediate release of Mn²⁺ in low pH environment and improve the dispersion of particles under neutral pH conditions.

The releasing rate of Mn²⁺ ions from MnO@10nm-SiO₂ was quantified at room temperature using 16 mg of MnO@10nm-SiO₂ suspension dispersed in 32 mL of citrate buffer at pH 5 or 6, or a distilled water. 4 mL of each sample was collected at 0 minute, 15 minutes, 1 hour, 2 hours, 4 hours, 6 hours, 8 hours and 12 hours and centrifuged at 15,000 rpm for 10 minutes to obtain nanoparticles (NPs) from the suspension. Then, the supernatant was filtered with a syringe filter of 0.2 µm (micro-meter) pore size. The concentration of Mn²⁺ ions in the filtered supernatant was measured by ICP-AES analysis and the releasing rate of Mn²⁺ ions was calculated using the mass of Mn²⁺ ion in the supernatant with respect to the mass of manganese in the sample.

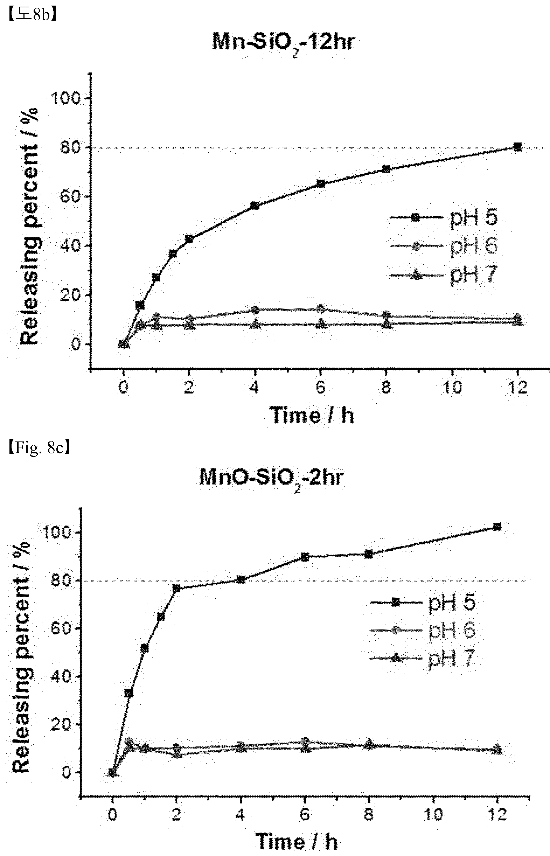

As shown in FIG. 8b, the nanoparticles having silica shell with 10 nm thickness slowly released Mn²⁺ in citrate buffer at pH 5 and took 12 hours to release 80 wt% of Mn²⁺ ions from MnO NPs. On the other hand, only 10 wt% of Mn²⁺ ions was released in Citrate buffer at pH 6.0 or distilled water at pH 7. Then, MnO@ SiO₂ NPs having silica shell with 10nm thickness were expressed as MnO@10nm-SiO₂ or MnO-SiO₂-12 hr (FIG. 8b).

### 8-2. Preparation of MnO@8nm-pSiO₂ (MnO-SiO₂-2hr) and measurement of Mn²⁺ releasing time

### (1) Production of MnO@8nm-pSiO₂ (MnO-SiO₂-2 hr) nanoparticles

To produce the particles that release Mn²⁺ ions faster than MnO@10 nm-SiO₂ at lower pH conditions, the porosity and thickness of the silica shell were adjusted.

For synthesizing the nanoparticles having silica shell with 8 nm thickness, 0.8 mL TEOS in Example 8-1 was replaced with 0.72 mL TEOS and 0.08 mL of C18TMS (n-octadecyl trimethoxy silane).

Next, to increase the porosity of the silica shell, an organosilane (C18TMS, n-octadecyltrimethoxy silane) was used as a porogen. The silane mixed with porogen (90 vol% TEOS + 10 vol% C18TMS) produces more pores in the silica shell, thereby allowing penetration of the solution into the core MnO.

### (2) Measurement of Mn²⁺ release time

The increased porosity or the decreased thickness of silica shell makes MnO nanoparticles more readily exposed to acidic solutions, thereby increasing the release rate of Mn²⁺.

As shown in FIG. 8c, MnO@SiO₂ NPs encapsulated with Porogen-mixed silane released 80 wt% of Mn²⁺ in 2 hours in citrate buffer solution at pH 5.0. The particles were then named as MnO@8nm-pSiO₂ or MnO-SiO₂-2hr.

MnO@10nm-SiO₂ and MnO@8nm-pSiO₂ NPs have similar thicknesses of silica shell, but shows very different release rate of Mn²⁺ in the citrate buffer at pH 5.0 due to the different porosity (FIG. 8c).

### 8-3. Production of MnO@5nm-SiO₂ (MnO-SiO₂-0.5hr)

### (1) Production of MnO@5nm-SiO₂ (MnO-SiO₂-0.5hr) nanoparticles

In order to reduce the thickness of the silica shell in the manufacturing method of Example 8-1, only half the amount (0.4 mL) of silica precursor (TEOS) was added as that of MnO@10nm-SiO₂ during the process of coating the MnO core with SiO₂. The silica shell thickness of the nanoparticles was 5 nm.

### (2) Measurement of Mn²⁺ release time

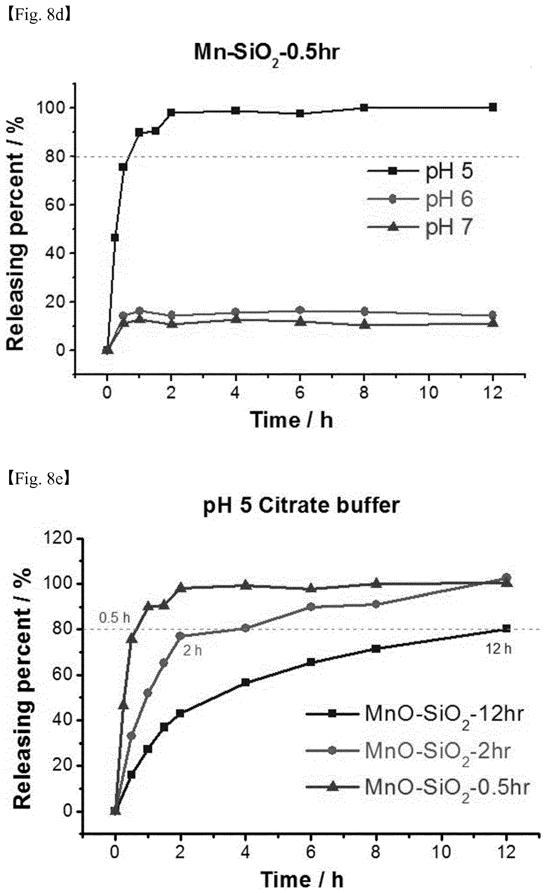

As shown in FIG. 8d, MnO@5nm-SiO₂ released 80 wt% of Mn²⁺ in 30 minutes at pH 5.0. Thereafter, the particles were named MnO@5nm-SiO₂ (Mn-SiO₂-0.5hr).

MnO@8nm-pSiO₂ and MnO@5nm-SiO₂ in Example 8-2 released only 10 wt% of Mn²⁺ in citrate buffer solution at pH 6.0 or distilled water (FIGs. 8c and 8d). For all designed nanoparticles, PEG end-capped with methoxy was used for efficient dispersion in aqueous conditions.

### 8-4. Comparison of difference in the release rate with the nanoparticle characteristics in similar pH condition of liver Kupffer cells

As measured in Examples 8-1 to 8-3, the release rate of Mn²⁺ ions was collected and compared for each particle under citrate buffer conditions at pH 5. The results are shown in FIG. 8e.

When the nanoparticles are injected into the body (in-vivo condition), they are absorbed by Kupffer cells in liver tissue. pH value inside the Kupffer cells is known to be about pH 5. In other words, in this experiment, the release rate of Mn²⁺ ions for each particle was predicted when the particles were absorbed into the Kupffer cells (in-vivo) by comparing the release rate of Mn²⁺ ions for each particle in-vitro condition of pH 5 buffer which is similar pH condition to that of Kupffer cell.

### 8-5. The contrast effect of released Mn²⁺ ion in T1-weighted MRI

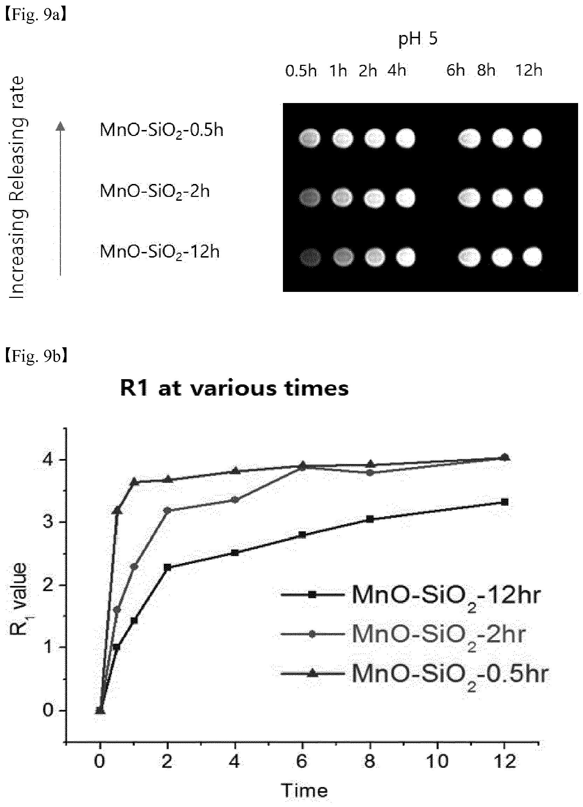

To determine what Mn²⁺ ions released from the nanoparticles exhibit the contrast effect on T1-weighted MRI, T1-weighted MRI of the supernatant solutions taken at the specific times for test the release rate of Mn²⁺ ions at citrate buffer (pH 5.0) in Example 8-1 was obtained and shown in Fig. 9a.

According to FIG. 9a, Mn²⁺ ions of MnO-SiO₂-0.5h particles were mostly emitted in 0.5 hour, and thus showed almost the same contrast intensity for 0.5h to 12h. Because the release rate of Mn²⁺ ions of MnO-SiO₂-12h particles was relatively low, the contrast intensity of MRI increased gradually over the time passage.

The contrast signal intensity of the photographed image was digitized (R1, unit= second (s)), and shown in the graph of FIG. 9b. It is inferred that the released Mn²⁺ ions can increase the contrast effect in T1-weighted image, on the basis that Fig. 9b is almost consistent with the graph of the release rate of Mn²⁺ ions for each particle shown in FIG. 8e.

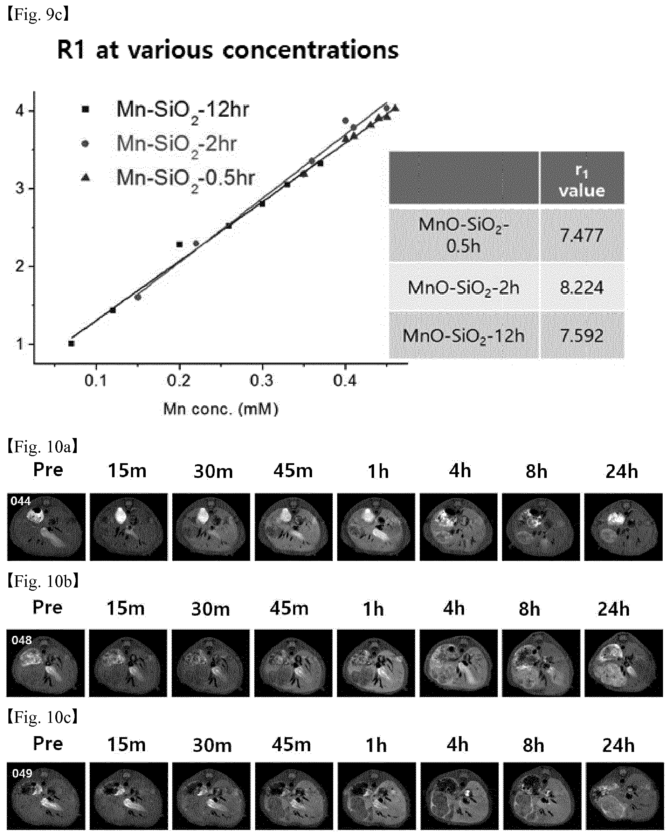

Next, the relationship between the concentration of Mn²⁺ ions and the contrast intensity R1 of the image is shown in the graph of FIG. 9c. The slope of this graph is expressed as r1, and is a unique value for each material. It was confirmed that the three particles had a similar slope within the range of 7.5 ∼ 8.2, and the increase in the contrast effect by Mn²⁺ ions was confirmed again by comparing with an r1 value of about 7.4 for pure Mn²⁺ ions (MnCl₂ aqueous solution).

### Example 9. In vivo MRI data

### 9-1. Production of HCC animal model

To test how MRI changes at different Mn²⁺ release rates of the designed MnO@SiO₂ contrast agent, the inventors inserted human hepatocellular carcinoma (HCC) into mice to produce an *in vivo* tumor model. Specifically, the manufacturing method of HCC animal model was in the same manner as in Example 1-4.

### 9-2. MRI imaging

Thereafter, MRI images of HCC were obtained in the same method of Example 1-5, except that MnO@5nm-SiO₂ (Mn-SiO₂-0.5hr) nanoparticles were used and the MRI images were collected at 15 minutes, 30 minutes, 45 minutes, 1 hour, 4 hours, 8 hours, and 24 hours after the nanoparticle injection.

### 9-3. MRI image changes after MnO@5nm-SiO₂ injection in HCC model

According to the method of Example 9-2, MRI image obtained after the injection of MnO@5nm-SiO₂ (MnO-SiO₂-0.5h) in the HCC model, and are shown in Fig. 10a.

### (1) The contrast change of normal liver tissue

As shown in FIG. 10a, when injecting MnO@5nm-SiO₂ into the HCC model, the liver parenchyma, a series of T1-weighted MRI contrast enhancement began to occur at 30 minutes and was maximized after 1 hour. After 1 hour, the contrast enhancement of the liver parenchyma gradually decreased and fully recovered to pre-injection level after 8 hours.

That is, MnO@5nm-SiO₂ NP reaching the liver through blood vessels was endocytosed into Kupffer cells and most of Mn²⁺ ions were released in 30 minutes, and thus the contrast enhancement began to occur. After 1 hour, Mn²⁺ ions were no longer released from Kupffer cells, and the contrast enhancement was decreased because Mn²⁺ ions absorbed by hepatocytes were released into biliary canaliculi through the hepatobiliary pathway.

### (2) The contrast change of HCC tumor tissue

HCC tumor tissue began to brighten from the peripheral area after 1 hour and the contrast enhancement gradually moves to the central area of HCC tissue (FIG. 10a). After 24 hours from the injection, the necrotic portion remained unchanged and dark contrast, while the contrast of the entire HCC tissue increased.

HCC tumor tissue lacks Kupffer cells and does not endocytose the nanoparticles directly. However, the hepatocytes of HCC tissues have mitochondrial activity, so that they can absorb Mn²⁺ released from Kupffer cells surrounding normal tissues. Therefore, the contrast enhancement of HCC tumor tissue begins at the peripheral area of the tumor and gradually brightens after about 1 hour when Kupffer cells in normal tissues release Mn²⁺ to the maximum level, and brightens up to the central region, as Mn²⁺ diffused and discharged from the parenchyma region surrounding the liver normal tissues moves to the inside of tumor tissue.

### (3) Criteria for distinguishing the liver normal tissue from HCC tumor tissue in case of MnO@5nm-SiO₂ contrast agent

Therefore, the normal hepatic tissue and HCC tumor tissue can be distinguished according to the brightness change pattern of contrast-enhanced image of the MRI image obtained over time after intravenous injection of MnO@5nm-SiO₂.

The normal tissue shows the high contrast enhancement for 30 minutes to 1 hour from the injection, and the low contrast enhancement for 1 to 24 hours. HCC tumor tissue was dark (black) or brightened in only peripheral region for 30 minutes to 4 hours from the injection time, and exhibited the high contrast enhancement for 4 hours to 24 hours.

### 9-4. MRI changes after MnO@8nm-pSiO₂ injection in HCC Model

According to the method of Example 9-2, MRI image obtained after the injection of MnO@8nm-pSiO₂ in the HCC model and are shown in Fig. 10b.

### (1) The contrast change of the liver parenchyma

The MRI image obtained by intravenous administration of MnO@8nm-pSiO₂ to the HCC model was not significantly different from the image obtained from MnO@5nm-SiO₂ (FIG. 10a) in Example 9-3.

In the normal liver parenchyma, the contrast enhancement began to increase in 30 minutes from the intravenous injection of MnO@8nm-pSiO₂ and reached the maximum level after 4 hours. However, unlike MnO@5nm-SiO₂, some contrast enhancement remained after 8 hours.

Like MnO@5nm-SiO₂, MnO@8nm-pSiO₂ also gradually increased the contrast of normal tissues. This is because Mn²⁺ release rate of nanoparticles endocytosed by the normal tissue is higher than Mn²⁺ release rate of the normal tissue. On the other hand, Mn²⁺ accumulated slowly in hepatocytes and remained later much than MnO@5nm-SiO₂. Thus, the maximum contrast enhancement occurred slowly and decreased slowly to the pre-injection level.

### (2)The contrast change of HCC tumor tissue

In HCC tumor tissue, the contrast did not increase until 1 hour from the nanoparticle injection, but began to increase from the peripheral region of tumor tissue after 4 hours and gradually brightened into inner part of the tumor until 24 hours.

The time required for the contrast enhancement in the peripheral region of HCC tumor tissue is determined by the release rate of Mn²⁺ ions from MnO NPs which are endocytosed by Kupffer cells located in normal tissue. Since MnO@8nm-pSiO₂ releases Mn²⁺ ions at a relatively lower release rate than MnO@5nm-SiO₂, the contrast enhancement in the peripheral region of HCC tumor tissue appeared slower than MnO@5nm-SiO₂.

### (3) Criteria for distinguishing the liver normal tissue from HCC tumor tissue in case of MnO@8nm-pSiO₂ contrast agent

Therefore, the normal liver and HCC tumor tissues can be distinguished according to the pattern of the brightness change of contrast-enhanced image over time for 45 minutes to 24 hours after intravenous injection of MnO@8nm-pSiO₂.

The normal tissues show the high contrast enhancement for 45 minutes to 4 hours, and the gradually decreased enhancement for 4 to 24 hours. HCC tumor tissues appear black for 45 minutes to 4 hours, but the contrast enhancement diffuses gradually from the outside with the high intensity into the inner area of tumor for 4 to 24 hours.

### 9-5. MRI changes after MnO@10nm-SiO₂ injection in HCC Model

According to the method of Example 9-2, MRI image obtained after the injection of MnO@10nm-SiO₂ in the HCC model are shown in Fig. 10c.

### (1) The contrast change of the liver parenchyma

As shown in FIG. 10c, unlike the previous two contrast agents of MnO@ 8nm-pSiO₂ and MnO@5nm-SiO₂, a series of T1-enhanced MRI between the liver parenchyma of HCC models controlled by MnO@10nm-SiO₂ began to produce the contrast enhancement after 45 min and the brightness was maintained to an equivalent level without significant change until 8 hours (FIG. 10c). After 24 hours, the contrast enhancement mediated by MnO@10nm-SiO₂ did not return to pre-injection level.

### (2)The contrast change of HCC tumor tissue

HCC tissue was black overall until 4 hours and the contrast enhancement in thin peripheral shape appeared after 8 hours. However, even after 24 hours, the contrast enhancement in the central portion of HCC tissue did not expand.

MnO@10nm-SiO₂ shows slower contrast enhancement even if Kupffer cells were endocytosed, since Mn²⁺ release rate is lower in acidic environment compared to the former two contrast agents. Since the rate of uptake of Mn²⁺ ions by HCC and the rate of Mn²⁺ ions release from Kupffer cells are at equilibrium, the contrast enhancement mediated by MnO@10nm-SiO₂ in normal tissues remains constant. The contrast enhancement of MnO@10nm-SiO₂ in the peripheral area showed the slowest of the three NPs.

### (3) Criteria for distinguishing the liver normal tissue from HCC tumor tissue in case of MnO@10nm-SiO₂ contrast agent

Therefore, the normal hepatic tissue and HCC tumor tissue can be distinguished according to the pattern of brightness change of contrast-enhanced image over time after the intravenous infusion of MnO@10nm-SiO₂ for 45 minutes to 24 hours.

The normal tissues show the high contrast enhancement for 45 minutes to 8 hours, and gradually decreased contrast enhancement for 8 to 24 hours. HCC tumor tissues appear dark (black) or have the contrast enhancement in only peripheral area for 45 minutes to 8 hours, and the high contrast enhancement in the overall tumor area for 8 to 24 hours.

### Example 10. Confirmation of long-term contrast enhancement after injection of contrast agent into HCC model

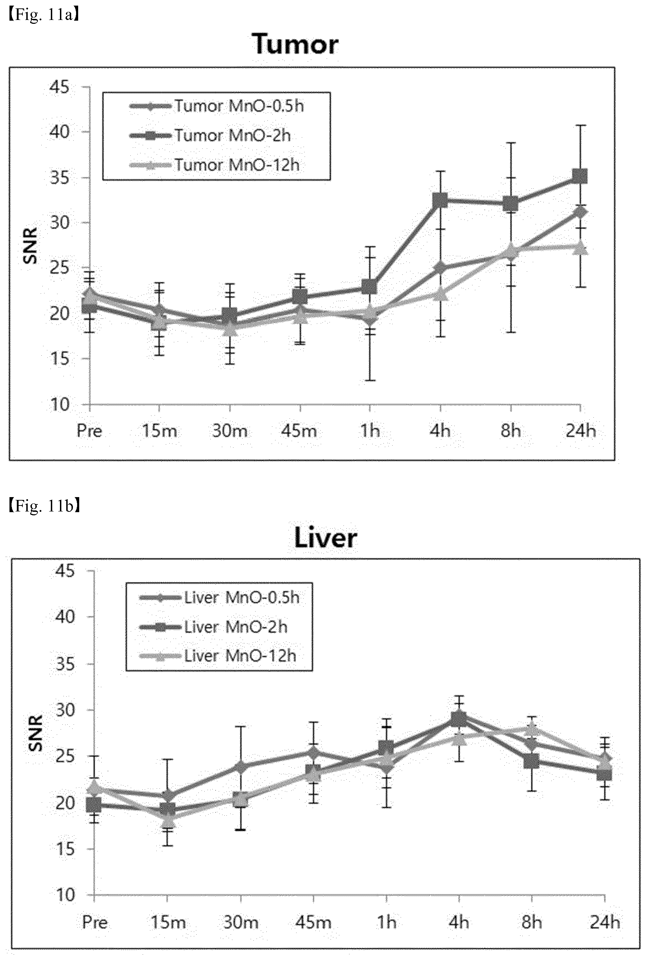

Each MnO@5nm-SiO₂, MnO@8nm-pSiO₂, and MnO@10nm-SiO₂ contrast agents synthesized in the previous Examples was administered to the primary liver tumor model (HCC model) according to the same method of Example 1-5, and then, the signals were measured on MRI images of each organ (paravision software version 5.0, Bruker-Biospin). The long-term contrast enhancement effect and discharge were measured by time, and the results are shown in Figs. 11a to 11e.

In the normal liver, the contrast was markedly marked after 30 minutes from the administration of MnO@5nm-SiO₂ (Mn-SiO₂-0.5hr) and was the strongest signal at 45 minutes, and gradually decreased after 1 hour. After 45 minutes from injection of MnO@8nm-pSiO₂ (Mn-SiO₂-2hr), the contrast effect was maintained, and the strongest signal was maintained from 1 hour to 4 hours and then decreased. After 45 minutes from injection of MnO@10nm-SiO₂ (Mn-SiO₂-12hr), the contrast effect appeared, and the contrast effect was maintained until 8 hours and decreased after 24 hours (FIG. 11a).

In the tumor area, the contrast effect of the normal region was decreased after 1 hour from the time of administration of MnO@5nm-SiO₂ (Mn-SiO₂-0.5hr), and the contrast effect of the tumor region was increased after 1 hour and maintained up to 24 hours. After 4 hours from the time of MnO@8nm-pSiO₂ (Mn-SiO₂-2hr) administration, the contrast effect of normal tissue was decreased, and then the contrast effect of the tumor tissue was increased (FIG. 11b). After 8 hours from the time of MnO@10nm-SiO₂ (Mn-SiO₂-12hr) administration the contrast effect of normal area was decreased, and then the contrast effect of tumor area was maintained for up to 24 hours (FIG. 11b).

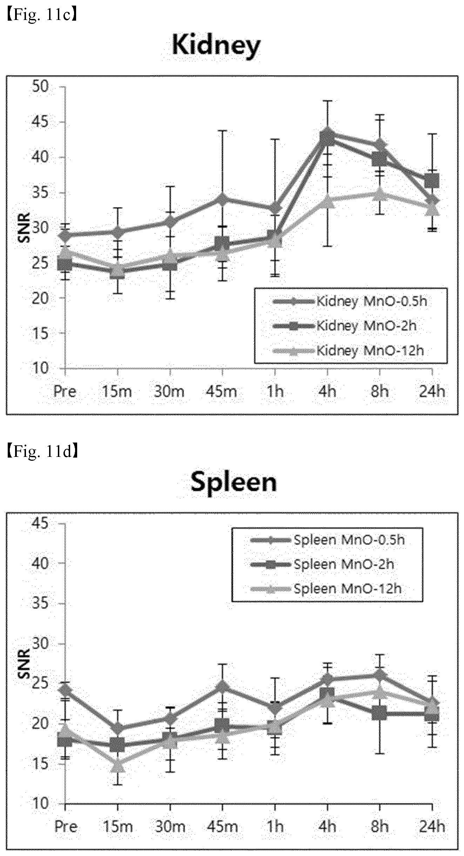

In case of kidney, after 4 hours from the time of administration of the contrast medium, the contrast effect in the kidney increased after 4 hours, which means that Mn²⁺ ions released through the renal pathway after 4 hours from the time of contrast agent administration (Fig. 11c).

No specific contrast effect was observed in the spleen (FIG. 11d).

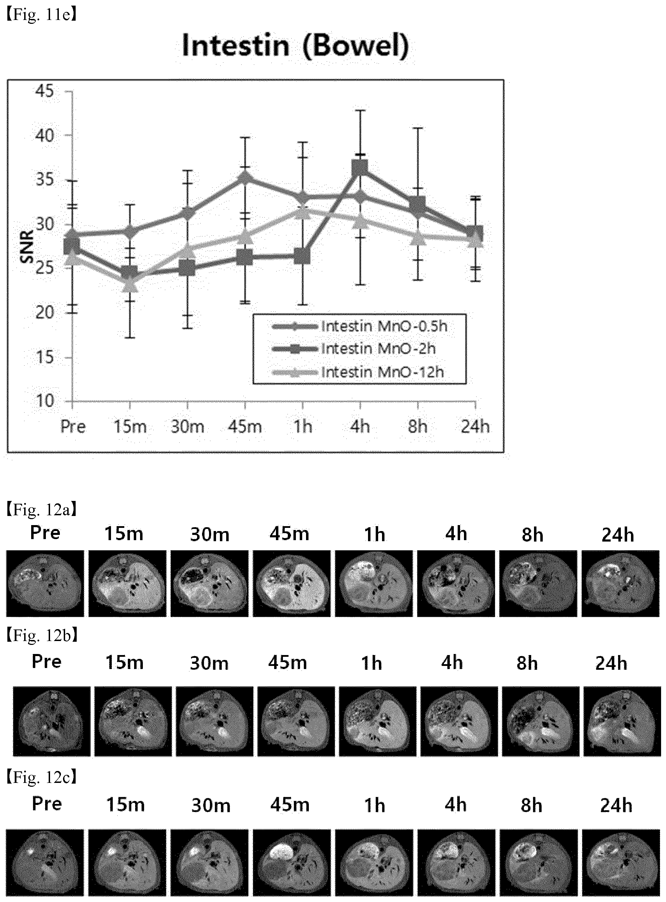

In the intestine, the contrast effect increased up to 45 min after the time of MnO@5nm-SiO₂ (MnO-SiO₂-0.5hr) administration, and then decreased. In case of MnO@8nm-pSiO₂ (MnO-SiO₂-2hr), the contrast effect was increased after 4 hours from the time of administration and then decreased. In the case of MnO@10nm-SiO₂ (MnO-SiO₂-12hr), the contrast effect was increased up to 1 hour after administration, and then was decreased (FIG. 11e).

### Example 11. Selection of the nanoparticles to control Mn²⁺ release rate and optimize the contrast at liver tumor type

In order to control the Mn²⁺ release rate, the present inventors conducted the experiments to determine the pathological characteristics and vascular angiogenesis of various tumor models (HCC, SNC, CAC) using three kinds of MnO₂@SiO₂ NPs prepared in Example 8. To do so, in vivo MRI results were obtained.

### 11-1. Production of animal model

In order to produce the tumor model of allogeneic xenograft mice, small intestinal neuroendocrine carcinoma (SNC) and colonic adenocarcinoma (CAC) as well as hepatocellular carcinoma (HCC) were used in the experiments as the representative non-hepatic cell metastatic tumor models of the cells having insufficient mitochondria and deficient mitochondria.

Specifically, SNC (STC-1) and CAC (HT29) animal models were prepared in the same manner as in Example 1-4.

### 11-2. MRI changes after the nanoparticle injection in CAC (HT29) Model

In accordance with the method of Example 9-2, MnO@5nm-SiO₂, MnO@8nm-pSiO₂, and MnO@10nm-SiO₂ were separately injected to the CAC model, and MRI images were obtained for 24 hours. MRI images are shown in Fig. 12a, Fig. 12b and Fig. 12c.

### (1) MnO@5nm-SiO₂ injection

The image obtained after injecting MnO@5nm-SiO₂ is shown in FIG. 12a. According to Fig. 12a, the normal tissue showed the contrast enhancement for 15 minutes to 45 minutes for the time of injection, the contrast enhancement gradually decreased from 1 hour to 24 hours and returned to the level before the injection of the contrast agent.

On the other hand, in the tumor tissue, the contrast enhancement was observed only at the rim area of the tumor for 15 minutes to 24 hours.

The contrast agent can be used to distinguish the normal tissue from CAC tumor tissue by analyzing the change pattern of MRI image over time. The normal tissue showed the high contrast for 15 to 45 minutes and gradually decreased from 1 hour to 24 hours. The tumor tissue remained black or only bright in the peripheral area for 15 minutes to 24 hours.

### (2) MnO@8nm-pSiO₂ injection

The MRI image obtained after injecting MnO@8nm-pSiO₂ is shown in FIG. 12b. According to FIG. 12b, the normal tissue showed the contrast enhancement for 30 minutes to 4 hours, and after 4 hours, the contrast enhancement gradually decreased and returned to the level before the injection of the contrast agent after 24 hours.

On the other hand, the contrast enhancement of tumor tissue began to increase from 45 minutes and from the edge of the tumor, and the two layers in the inner part of the tumor were formed in which the inner layer was black with no contrast enhancement, and the outer layer directly surrounding the black portion had some contrast enhancement spread inward like HCC.

The contrast agent can be used to distinguish the normal tissue from CAC tumor tissue by analyzing the change pattern of MRI image over time. The normal tissue shows the high contrast enhancement for 30 minutes to 4 hours, and the gradually decreased contrast enhancement after 4 hours to 24 hours. The tumor tissue remained black or only bright in the peripheral region for 30 minutes to 24 hours.

### (3) MnO@10nm-SiO₂ injection

The MIR image obtained after injecting MnO@10nm-SiO₂ is shown in FIG. 12c. According to Fig. 12c, the normal tissue showed the contrast enhancement for 15 minutes to 4 hours, and the contrast enhancement gradually decreased for 8 hours to 24 hours and returned to the level before the contrast injection.

On the other hand, in the tumor tissues, only the edge of the tumors was continuously enhanced from 1 hour to 24 hours.

The contrast agent can be used to distinguish the normal tissue from CAC tumor tissue by analyzing the change pattern of MRI image over time. The normal tissue showed the high contrast enhancement for 15 minutes to 4 hours and the decreased contrast enhancement from 8 hours to 24 hours. The tumor tissue appeared black or only bright in the peripheral region for 15 minutes to 4 hours, and the contrast enhancement was observed in only the borders of tumors for 8 to 24 hours.

### 11-3. MRI image changes after nanoparticle injection in SNC (STC-1) Model

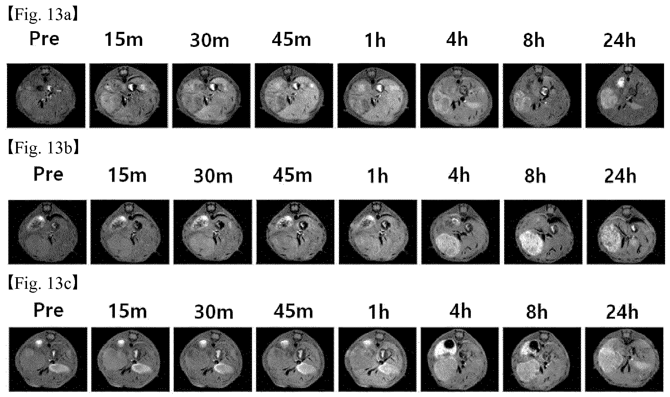

According to the method of Example 9-2, MRI images over time obtained for 24 hours after MnO@5nm-SiO₂, MnO@8nm-pSiO₂, and MnO@10nm-SiO₂ were separately injected in the SNC model. MRI images are shown in Fig. 13a, Fig. 13b and Fig. 13c.

### (1) MnO@5nm-SiO₂ injection

The MRI image obtained after injecting MnO@5nm-SiO₂ is shown in FIG. 13a. According to FIG. 13a, the normal tissue showed the contrast enhancement for 15 minutes to 4 hours, and contrast enhancement gradually decreased for 4 hours to 24 hours, and returned to the level before the injection of contrast agent.

On the other hand, the tumor tissues appeared similar to the normal tissues for 30 minutes to 1 hour, but the contrast enhancement continued for 4 to 24 hours.

The contrast agent can be used to distinguish between the normal tissue and SNC tumor tissue by analyzing the change pattern of MRI image over time, and the two tissues are not distinguished well for 15 minutes to 1 hour, but the contrast enhancement in the tumor tissue gradually appeared and brightened after 4 hours up to 24 hours.

### (2) MnO@8nm-pSiO₂ injection

The MRI image obtained after injecting MnO@8nm-pSiO₂ is shown in FIG. 13b. According to FIG. 13b, the normal tissue showed the contrast enhancement for 15 minutes to 1 hour with no high intensity, and the contrast enhancement was not observed for 4 to 24 hours.

The tumor tissue, on the other hand, appeared darker at lower intensity from 15 minutes to 1 hour after the contrast agent injection, but appeared brighter than the normal tissue, and continued to brighten at high intensity for 4 to 24 hours.

The contrast agent can be used to distinguish the normal tissue from SNC tumor tissue by analyzing the change pattern of MRI image over time, and it is difficult to clearly distinguish between the normal tissue and the tumor tissue for 15 minutes to 1 hour. From 4 hours to 24 hours, the normal tissues show the low contrast enhancement and the tumor tissues show the high contrast enhancement.

### (3) MnO@10nm-SiO₂ injection

The MRI image obtained after injecting MnO@10nm-SiO₂ is shown in FIG. 13c. According to FIG. 13c, the normal tissues showed the contrast enhancement with no high intensity for 30 minutes to 1 hour, and no contrast enhancement was observed for 4 hours to 24 hours.

On the other hand, the tumor tissues showed the low contrast enhancement for 15 minutes to 1 hour, but were stronger than the normal tissues, and continued to increase the contrast intensity from 4 hours to 24 hours.

The contrast agent was able to distinguish between the normal tissue and SNC tumor tissue by analyzing the change pattern of MRI image over time, and it is not easy to distinguish between the two tissues for 15 minutes to 1 hour, but the normal tissue showed the low contrast enhancement, but and tumor showed the high contrast enhancement for 4 hours to 24 hours.

### 11-4. Test of the optimal nanoparticles for screening HCC, CAC and SNC tumors

The contrast enhancement patterns of the SNC and CAC models were similar to that of the HCC model only in the normal tissue area. Regardless of the release rate of Mn²⁺, the contrast enhancement began when MnO@SiO₂ nanoparticles were absorbed by Kupffer cells in normal tissues.

HCC has excessive blood vessels, high mitochondrial activity and characteristic release pathways. Because of the high mitochondrial activity of HCC, the uptake of Mn²⁺ released from Kupffer cells effectively occurs around normal cells. In addition, because the peripheral excretion by the HCC pathway also occurs inside the HCC, the contrast enhancement in the peripheral region is observed with slower than that of the SNC.

As a result, MnO@10nm-SiO₂ is the most effectively optimized MRI contrast agent in HCC diagnostic systems. When using MnO@5nm-SiO₂, due to the rapid enhancement of the normal tissue, MRI of HCC is darkened between 45 minutes and 1 hour, thereby being capable of characterizing. On the other hand, the MRI of the normal tissues is restored after 24 hours, while the contrast in the MRI of HCC is enhanced to diagnose liver cancer again.

SNC has many blood vessels and high mitochondrial activity, but no release pathway. Because of the high mitochondrial activity, Mn²⁺ provided by Kupffer cells is effectively absorbed by surrounding normal tissues, but Mn²⁺ accumulates continuously due to no excretion pathway. These results in a faster overall contrast enhancement for SNC compared to HCC. Engineered MnO@SiO₂ nanoparticles are endocytosed by Kupffer cells, and SNC tumor tissue and normal tissues show the contrast enhancement almost simultaneously. Thus, MnO@5nm-SiO₂ and MnO@8nm-pSiO₂ with fast recovery rates are the most effective contrast agents in the SNC tumor model system.

CAC is hypovascular and does not have a mitochondrial release pathway. Because of the deficient mitochondria, the uptake of Mn²⁺ for the entire measurement period is not effective. This only shows the contrast enhancement of the thin layer in the peripheral region. MnO@5nm-SiO₂ represents the maximum contrast enhancement in the normal tissues, and thus the marked contrast between the CAC portion and normal tissues is observed. On the other hand, MnO@10nm-SiO₂ can be used to analyze slow rate procedures, since the normal tissue maintains slightly improved contrast with no noticeable change for a long time after 1 hour after NP injection.

CAC model system shows slower contrast behavior than normal cells, because Mn²⁺ ions are ingested after Mn²⁺ ions are released from the surrounding normal tissues. Because of the high mitochondrial activity of HCC and SNC, it is beneficial to use the nanoparticles that quickly recover to pre-injection levels for a clear comparison between the tumor and normal tissues. On the other hand, in the CAC, it is advantageous to use MnO@10nm-SiO₂ because of the extended contrast enhancement.

In summary, the analysis of the change pattern of MRI contrast enhancement over time obtained using designed MRI contrast agents can not only differentiate tumors from the normal liver tissues, but also characterize and discriminate the tumor types and origins in body.

## Claims

1. A liver tissue-specific magnetic resonance imaging (MRI) contrast agent, comprising manganese silicate nanoparticles capable of releasing manganese ions under acidic conditions, wherein the manganese ions are specifically accumulated in a liver tissue to produce a specifically distinguishable change pattern of MRI.

2. The liver tissue-specific MRI contrast agent according to claim 1, wherein the manganese-silicate nanoparticles are absorbed into Kupffer cells of liver tissue to release manganese ions, and the released manganese ions are excreted outside the Kupffer cells and specifically absorbed and accumulated in the liver tissue, to produce the MRI change pattern that is specifically differentiated in the liver tissue.

3. The liver tissue-specific MRI contrast agent according to claim 2, wherein the release rate of manganese ions released from the outside of Kupffer cells is such that the manganese ions accumulate specifically in the liver tissue, to produce the MRI change pattern that is specifically differentiated in the liver tissue.

4. The liver tissue-specific MRI contrast agent according to claim 1, wherein the contrast agent generates MRI change pattern over time, which is specifically distinguished in the liver tissue.

5. The liver tissue-specific MRI contrast agent according to claim 1, wherein the contrast agent analyzes vascularity, cell density, mitochondrial activity, or hepatocellular affinity.

6. The liver tissue-specific MRI contrast agent according to claim 3, wherein the release rate of manganese ions is controlled by regulating the porosity and the layer thickness of nanoparticles.

7. The liver tissue-specific MRI contrast agent according to claim 1, wherein the contrast agent is a bright contrast agent for T₁-weighted MRI.

8. The liver tissue-specific MRI contrast agent according to claim 1, wherein the contrast agent distinguishes a liver tumor from a normal liver tissue.

9. The liver tissue-specific MRI contrast agent according to claim 1, wherein the contrast agent differentiates the type of liver tumors.

10. The liver tissue-specific MRI contrast agent according to claim 1, wherein the contrast agent monitors the therapeutic effect of liver tumor treatment.

11. The liver tissue-specific MRI contrast agent according to claim 1, wherein the manganese-silicate nanoparticles have a hollow structure.

12. The liver tissue-specific MRI contrast agent according to claim 1, wherein the manganese-silicate nanoparticles have a structure including core of manganese-oxide and shell of silica.

13. The liver tissue-specific MRI contrast agent according to claim 1, wherein the manganese-silicate nanoparticles comprise manganese ions in an amount of 0.5-55% by weight based on the total weight of the nanoparticles.

14. The liver tissue-specific MRI contrast agent according to claim 8, wherein the contrast agent generates MRI change that is specific to disease over time *in vivo.*

15. The liver tissue-specific MRI contrast agent according to claim 14, wherein the MRI change pattern is a brightness change pattern in MRI.

16. The liver tissue-specific MRI contrast agent according to claim 15, wherein the MRI change pattern over time obtained after administration of the contrast agent has a pattern in which is gradually brightened, and after reaching the maximum brightness, is gradually darken in normal liver tissue.

17. The liver tissue-specific MRI contrast agent according to claim 15, wherein the liver tissue is hepatocellular carcinoma (HCC), and
the MRI change pattern of hepatocellular carcinoma tissue over time obtained after the administration of the contrast agent has a pattern in which the hepatic cell carcinoma is maintained in a dark state after administering the contrast agent and begins to brighten at the time that the normal liver tissue reaches the maximum brightness, and then maintains the brightness over time, on the basis of the MRI change pattern of the normal liver tissue which is gradually brightened and after reaching the maximum brightness, is gradually darken.

18. The liver tissue-specific MRI contrast agent according to claim 15, wherein the liver tissue is colon adenocarcinoma (CAC), and
the MRI change pattern of colon adenocarcinoma over time obtained after the administration of the contrast agent has a pattern in which the CAC is maintained in a dark state, is gradually brightened in darker state than the normal liver tissue, or has a bright circle only in the rim over time after administering the contrast agent, on the basis of the MRI change pattern of the normal liver tissue which is gradually brightened and after reaching the maximum brightness, is gradually darken.

19. The liver tissue-specific MRI contrast agent according to claim 15, wherein the liver tissue is a small intestinal neuroendocrine carcinoma (SNC) and
the MRI change pattern of SNC over time obtained after the administration of the contrast agent has a pattern in which the SNC is gradually brightened and maintained in the brighter state than the normal liver tissue, without being darkened over time after administering the contrast agent, on the basis of the MRI change pattern of the normal liver tissue which is gradually brightened and after reaching the maximum brightness, is gradually darken.

20. A method of characterizing the liver tissue, comprising administering one or more of the contrast agents of claim 1 to claim 19 to a subject; obtaining MRI by continuously imaging the liver tissue of the subject over time; and extracting a temporal change pattern of MRI of the liver tissue.

21. The method of characterizing the liver tissue according to claim 20, wherein the MRI change pattern of the liver over time is a brightness change pattern of MRI.

22. The method of characterizing the liver tissue according to claim 20, wherein in the MRI obtained by continuously imaging the liver tissue of the subject over time after administering the contrast agent, the normal liver tissue has a pattern in which is gradually brightened and after reaching the maximum brightness, is gradually darken.

23. The method of characterizing the liver tissue according to claim 20, wherein the liver tissue is hepatocellular carcinoma (HCC), and
the MRI change pattern of hepatocellular carcinoma over time obtained after administering the contrast agent has a pattern in which the hepatic cell carcinoma is maintained in a dark state over time after administering the contrast agent and then begins to brighten at the time that the normal liver tissue reaches the maximum brightness and then maintains the brightness, on the basis of the MRI change pattern of the normal liver tissue which is gradually brightened and after reaching the maximum brightness, is gradually darken.

24. The method of characterizing the liver tissue according to claim 20, wherein the liver tissue is colon adenocarcinoma (CAC), and
the MRI change pattern of colon adenocarcinoma over time obtained after administering the contrast agent has a pattern in which the CAC is maintained in a dark state, is gradually brightened in darker state than the normal liver tissue, or has a bright circle only in the rim over time after administering the contrast agent, on the basis of the MRI change pattern of the normal liver tissue which is gradually brightened and after reaching the maximum brightness, is gradually darken.

25. The method of characterizing the liver tissue according to claim 20, wherein the liver tissue is a small intestinal neuroendocrine carcinoma (SNC) and
the MRI change pattern of SNC over time obtained after the administration of the contrast agent has a pattern in which the SNC is gradually brightened and maintained in the brighter state than the normal liver tissue, without being darkened over time after administering the contrast agent, on the basis of the MRI change pattern of the normal liver tissue which is gradually brightened and after reaching the maximum brightness, is gradually darken.

26. A method for providing information on the diagnosis of liver tumor and the determination of type of liver tumors, comprising administering one or more contrast agent according to claim 1 to claim 19 to a subject, and obtaining a MRI by continuously imaging the liver tissue of the subject over time.
